# EUROPÄISCHE PATENTSCHRIFT

(11) **EP 1 444 358 B1**
(45) Veröffentlichungstag und Bekanntmachung des Hinweises auf die Patenterteilung: **15.07.2009**
(21) Anmeldenummer: 02785384.5
(22) Anmeldetag: 12.11.2002
(51) Int. Cl.: C12Q 1/18

(54) **VERFAHREN ZUR CHARAKTERISIERUNG UND/ODER IDENTIFIZIERUNG VON WIRKMECHANISMEN ANTIMIKROBIELLER PRÜFSUBSTANZEN**
METHOD FOR CHARACTERISING AND/OR IDENTIFYING ACTIVE MECHANISMS OF ANTIMICROBIAL TEST SUBSTANCES
PROCEDE DE CARACTERISATION ET/OU D'IDENTIFICATION DE MECANISMES D'ACTION DE SUBSTANCES D'ESSAI ANTI-BACTERIENNES

(30) Priorität: 12.11.2001 DE 10155185
(43) Veröffentlichungstag der Anmeldung: 11.08.2004
(73) Patentinhaber: Synthon KG, 69118 Heidelberg (DE)
(72) Erfinder: LABISCHINSKI, Harald, 42113 Wuppertal (DE); SCHIFFER, Guido, D- 42111 Wuppertal (DE); SCHMITT, Jürgen, 69118 Heidelberg (DE); UDELHOVEN, Thomas, 54296 Trier (DE)
(74) Vertreter: Huhn, Michael
(86) Internationale Anmeldenummer: PCT/EP2002/012642
(87) Internationale Veröffentlichungsnummer: WO 2003/042406

(56) Entgegenhaltungen:
- WO-A-91/05238
- WO-A-94/11526
- DE-A- 3 003 778
- D. NAUMANN: "Infrared and NIR Raman Spectroscopy in Medical Microbiology" PROCEEDINGS OF SPIE, H.H. MANTSCH AND M. JACKSON (EDS.), [Online] Bd. 3257, - 1998 Seiten 245-257, XP002238946 Bellingham, Washington Gefunden im Internet: <URL:midas.rki.de/publications/nau_spie.pd f> [gefunden am 2003-04-22]
- THI N A N ET AL: "FT-IR microspectrometry: a new tool for characterizing micro-organisms" BIOMEDICAL SPECTROSCOPY: VIBRATIONAL SPECTROSCOPY AND OTHER NOVEL TECHNIQUES, SAN JOSE, CA, USA, 26-27 JAN. 2000, Bd. 3918, Seiten 36-44, XP002238947 Proceedings of the SPIE - The International Society for Optical Engineering, 2000, SPIE-Int. Soc. Opt. Eng, USA ISSN: 0277-786X
- ZEROUAL WIDAD ET AL: "FT-IR spectroscopy study of perturbations induced by antibiotic on bacteria (Escherichia coli)." PATHOLOGIE BIOLOGIE, Bd. 43, Nr. 4, 1995, Seiten 300-305, XP009009734 ISSN: 0369-8114
- C. KIRSCHNER ET AL.: "FT-IR Spectroscopic Investigations of Antibiotic Sensitive and Resistant Microorgansims" POSTER: 8TH EUROPEAN CONFERENCE ON THE SPECTROSCOPY OF BIOLOGICAL MOLECULES, ENSCHEDE, THE NETHERLANDS, [Online] 29. August 1999 (1999-08-29) - 2. September 1999 (1999-09-02), Seite 1 XP002238948 Gefunden im Internet: <URL:midas.rki.de/publications/kir_poster1 .pdf> [gefunden am 2003-04-22]
- D. NAUMANN: "Biophysikalische Strukturanalysen" ROBERT KOCH INSTITUT FORSCHUNGSPROJEKTE PROF. DR. D. NAUMANN, [Online] - 2000 Seiten 1-4, XP002238949 Gefunden im Internet: <URL:www.rki.de/FORSCH/PG/P34.HTM> [gefunden am 2003-04-17]
- MASON DAVID J ET AL: "An inexpensive infrared growth sensor array for detection of bacterial antibiotic susceptibility." FEMS (FEDERATION OF EUROPEAN MICROBIOLOGICAL SOCIETIES) MICROBIOLOGY, Bd. 111, Nr. 2-3, 1993, Seiten 251-254, XP009009735 1993 ISSN: 0378-1097

## Beschreibung

Die Erfindung betrifft ein Verfahren zur Charakterisierung und/oder Identifizierung von Wirkmechanismen insbesondere antimikrobiell wirksamer Prüfsubstanzen mit Hilfe von IR (Infrarot)-, FT-IR (Fourier-Transform-Infrarot)-, Raman- oder FT-Raman (Fourier-Transform Raman)- Analysen.

Epidemiologische Studien belegen, daß die Resistenzraten von pathogenen Mikroorganismen, wie z.B. bakteriellen Keimen gegen handelsübliche Hemmstoffe und Inhibitoren wie z.B. Antibiotika, Antimykotika und andere Chemotherapeutika im Verlauf der letzten zwei Jahrzehnte ständig zugenommen haben [Levy S.B. (2001) Antibiotic resistance: consequences of inaction. Clin Infect Dis Sep 15;33 Suppl 3: pp. 124-9]. Um in diesem Umfeld steigender Resistenzraten gegen bekannte Antibiotika die Therapierbarkeit bakterieller Infektionen auch in Zukunft zu gewährleisten, werden weltweit große Anstrengungen unternommen, neue Leitstrukturen für die Antibiotikatherapie zu identifizieren und zu entwickeln. Dabei ist die Untersuchung des Wirkmechanismus derartiger neuer Leitstrukturen für die Erforschung und Entwicklung antimikrobieller Substanzen von zentraler Bedeutung. Unter dem Terminus Wirkmechanismus (Targetaufklärung) wird dabei die Identifizierung der Stoffwechselwege bis hin zu molekularen Einzelvorgängen verstanden, die in kausalem Zusammenhang mit der antimikrobiellen Wirkung einer neuen Leitstruktur stehen. Einerseits erlaubt die Kenntnis der mikrobiellen Zielstruktur, des Targets, eine schnelle und effiziente Optimierung der Leitstruktur *in vitro,* z.B. in einem subzellulären Target-Assay; andererseits können potentielle toxikologische Nebenwirkungen, die auf der Inhibierung eines möglicherweise im Wirt ebenfalls vorkommenden homologen Targets beruhen, bereits frühzeitig in einem entsprechenden Gegentest erkannt werden. Bei Kenntnis des molekularen Targets bzw. des Targetgebiets kann darüber hinaus die Entwicklung einer antimikrobiellen Prüfsubstanz mit unselektivem Wirkmechanismus (z.B. allgemein membranzerstörende Detergenzwirkung, Zerstörung des Membranpotentials durch Ionophore, Interkalation in Nukleinsäuren) verhindert werden, wodurch u.a. Forschungskosten eingespart werden können.

Heutige in der Humantherapie eingesetzte Antibiotika zeichnen sich durch eine spezifische Wirkung auf einen für die Lebensfähigkeit des Bakteriums essentiellen Stoffwechselweg aus (vergl. z.B. Graefe U. (1992) Biochemie der Antibiotika, pp. 15-39, Spektrum Akademischer Verlag Heidelberg, Berlin, New York). Die überwiegende Zahl der bisher bekannten Antibiotikaklassen inhibitiert oder dereguliert die Biosynthese von bakteriellen Makromolekülen wie z.B. DNA (Beispiele: Chinolone, Novobiocin), RNA (Beispiele: Rifampicin, Streptolydigin, Lipiarmycin, Holomycin), Protein (Beispiele: Makrolide/Ketolide, Aminoglykoside, Tetracycline, Oxazolidinone) oder Peptidoglykan (Beispiele: β-Lactame, Fosfomycin, Vancomycin, Moenomycin). Andere Antibiotika üben ihre Wirkung über die Hemmung von Stoffwechselwegen des Intermediärmetabolismus aus (z.B. Sulfonamide und Trimethoprim als Inhibitoren des C₁-Stoffwechsels; Cerulenin als Inhibitor der Fettsäurebiosynthese).

Häufig kann die antibiotische Wirkung direkt auf die Inhibierung eines definierten Enzyms oder einer Enzymfamilie zurückgeführt werden; beispielsweise hemmen β-Lactame irreversibel die Enzymfamilie der für die Zellwandsynthese unabdingbaren Penicillin-Bindeproteine und leiten damit letztlich die Autolyse der Bakterienzelle ein. In anderen Fällen dienen größere, makromolekulare Strukturen wie z.B. Ribosomen - Ribonukleoproteinkomplexe, die die Translation der mRNA in eine Proteinsequenz katalysieren - als Angriffsorte von Antibiotika (z.B. Makrolide) (Graefe U. (1992) Biochemie der Antibiotika, pp. 15-39, Spektrum Akademischer Verlag Heidelberg, Berlin, New York, Russell A.D., Chopra I. (1996) Understanding Antimicrobial Action and Resistance, 2nd edition, pp. 28-83, Ellis Horwood, London).

Nach dem bisherigen Stand der Technik kommen zur Klärung des Wirkmechanismus von antimikrobiell aktiven Substanzen insbesondere folgende Methoden einzeln oder kombiniert zum Einsatz:
1) Im Absterbeexperiment (Rybak M.J. et al. (2000) In vitro activities of daptomycin, vancomycin, linezolid, and quinupristin-dalfopristin against Staphylococci and Enterococci, including vancomycin- intermediate and -resistant strains. Antimicrob. Agents Chemother. 44(4):1062-1066) wird die Anzahl der überlebenden Bakterien in Abhängigkeit von der Einwirkungsdauer der zu testenden Substanz im Vergleich zu einer unbehandelten Kontrollkultur unter ansonsten gleichen Wachstumsbedingungen bestimmt. Dies erlaubt jedoch nur eine grobe Unterscheidung zwischen bakteriostatischen (wachstumshemmenden) und bakterioziden (abtötenden) Substanzen.
2) Im Metaboliteneinbautest (Oliva B. et al. (2001) Antimicrobial properties and mode of action of the pyrrothine holomycin. Antimicrob. Agents Chemother. 45, pp. 532-539) werden bakterielle Zellen unter geeigneten Kulturbedingungen in Gegenwart der zu testenden Leitstruktur mit solchen radioaktiven Vorstufen für wichtige Stoffwechselwege (z.B. [¹⁴C]-Thymidin, [¹⁴C]-Uridin, [¹⁴C]-Leucin, [¹⁴C]-N-Acetylglukosamin) inkubiert, welche selektiv in hochmolekulares, mit Säuren oder organischen Lösungsmitteln fällbares Material (DNA, RNA, Protein, Peptidoglykan) eingebaut werden. Nach Trennung des hoch- vom niedermolekularen, löslichen Anteils der Radioaktivität durch Filtration oder Zentrifugation stellt die Radioaktivität in der hochmolekularen Fraktion ein Maß für die Syntheseleistung der Zelle in dem jeweiligen Stoffwechselweg dar. Dieser Test ist automatisierbar (Renick P. J. and Morris, T. W. (2000) Simultaneous parallel assays for inhibition of major metabolic pathways in intact cells of Staphylococcus aureus. Poster F-2023 Session 211, 40th Interscience Conference on Antibacterial Agents and Chemotherapy, Toronto), erfasst jedoch ausschließlich Targets im Bereich der Makromolekülbiosynthese. Targets im Bereich des Intermediärstoffwechsels werden in der Regel nicht erfasst. Eine weitere Einschränkung des Verfahrens ist die Abhängigkeit von der Verfügbarkeit radioaktiv markierter, selektiver Vorstufen.
3) Bei den genetischen Methoden (Zhang L. et al. (2000) Regulated gene expression in Staphylococcus aureus for identifying conditional lethal phenotypes and antibiotic mode of action. Gene 255(2):297-305) wird vor allem die Konstruktion von Über- oder Unterexpressionsmutanten (einzelne Stämme oder Mutantenbibliotheken) angewandt, die häufig zu einer Änderung der Empfindlichkeit gegenüber der zu testenden Leitstruktur führen, sofern die Mutation ein Gen des betroffenen Stoffwechselweges betrifft. Eine weitere Vorgehensweise besteht in der Selektion von Mutanten, die gegen die Testsubstanz resistent sind. Von der genomischen DNA dieser Mutanten kann eine Vektorbibliothek auf Basis von z.B. Plasmiden, Cosmiden, Bakteriophagen u.a. hergestellt werden. Mit Hilfe gängiger molekulargenetischer und mikrobiologischer Techniken ist es möglich, die Mutation zu identifizieren und damit das potentielle Target einzugrenzen oder ein mit dem Target in Beziehung stehendes Gen zu zu identifizieren. Diese Methoden sind sehr arbeitsintensiv, nicht automatisierbar und damit sehr zeitaufwendig und ressourcenintensiv.
4) Bindungsexperimente zum direkten Nachweis der Bindung der zu testenden Leitstruktur an ihr Target geben häufig sehr direkte Hinweise auf den Wirkmechanismus (Spratt B.G. (1977) Properties of penicillin-binding proteins of Escherichia coli K12. Eur. J. Biochem. 72:341-352). Dabei wird die Tatsache ausgenutzt, dass die Wechselwirkung zwischen antimikrobiell wirksamen Substanzen und ihren Wirkorten in der Regel sehr stark sind (z.B. kovalente Bindung im Fall von Beta-Lactamen) und somit häufig analytische Manipulationen zur Isolierung und Detektion des Target - Inhibitor Komplexes überstehen. Nachteilig ist jedoch, dass dazu in der Regel geeignet (z.B. radioaktiv) markierte Inhibitoren zur Verfügung stehen müssen, die häufig nicht oder nur mit erheblichem Aufwand erhältlich sind und besonders im Falle schwächerer, nicht-kovalenter Interaktionen der gewünschte Komplex nicht isoliert werden kann. Hinzu kommt, dass für jeden Einzelfall ein neu angepasstes Verfahren zu etablieren ist (z.B. in Abhängigkeit von der subzellulären Lokalisation des Targets, von der Natur der Bindung zwischen Target und Inhibitor).

Die beschriebenen Methoden zur Targetcharakterisierung haben den Nachteil, daß sie wie im Fall des Absterbeexperiments nur einen geringen Informationsgehalt liefern oder nicht allgemein und einheitlich auf verschiedenste Targetgebiete anwendbar sind und darüber hinaus sehr viel Zeit in Anspruch nehmen. Die Aufklärung des Wirkmechsnismus kann sich in Einzelfällen über mehrere Jahre erstrecken. So ist selbst 14 Jahre nach der ersten Beschreibung des Daptomycins (Allen N. E. et al. (1987) Inhibition of peptidoglycan biosynthesis in gram-positive bacteria by LY146032. Antimicrob. Agents Chemother. 31, 1093-1099) der molekulare Wirkmechanismus immer noch nicht vollständig aufgeklärt.

WO 91/05238 betrifft ein Verfahren zur Charakterisierung von Mikroorganismen. Als Ausgangspunkt der Kategorisierung werden spektrale Eigenschaften des Mikroorganismus verwendet. Als weitere Parameter, die zur Klassifizierung des Mikroorganismus dienen, wird die Sensitivität gegenüber Antibiotika verwendet. Das Antibiotika wird als Mittel verwendet, um eine Reaktion des Mikroorganismus, anhand dem die Spektraleigenschaften des Mikroorganismus untersucht und somit der Mikroorganismus klassifiziert wird.

D. NAUMANN: "Infrared and NIR Raman Spectroscopy in Medical Microbiology", PROCEEDINGS OF SPIE, H.H. MANTSCH AND M., JACKSON (EDS.), vol. 3257, - 1998 pages 245-257, betrifft die Untersuchung von intakten Zellen mittels FT-IR und FT-Raman-Analyse. Der spektrale Fingerabdruck wird verwendet, um Aussage über den untersuchten Mikroorganismus zu treffen. Zur Unterscheidung der Spektren der Mikroorganismen wird eine multivariante statistische Analyse oder ein neuronales Netz verwendet.

N. A. NGO THI et. al. "FT-IR microspectrometry: a new tool for characterizing microorganisms", BIOMEDICAL SPECTROSCOPY: VIBRATIONAL SPECTROSCOPY AND OTHER NOVEL TECHNIQUES, SAN JOSE, CA, USA, 26-27 JAN. 2000, vol. 3918, Seiten 36-44, 2000, SPIE-Int. Soc. Opt. Eng, USA, beschreibt ebenso die Spektraluntersuchung von Mikrorganismen. Oxazillin-behandelte Kulturen werden mit unbehandelten Kulturen verglichen, um die Kulturen näher zu untersuchen.

ZEROUAL WIDAD ET AL: "FT-IR spectroscopy study of perturbations induced by antibiotic on bacteria (Escherichia coli)." PATHOLOGIE BIOLOGIE, Vol. 43, Nr.. 4, 1995, Seiten 300-305, beschreibt FT-IR Spektroskopie zur Analyse von Antibiotikaauswirkungen auf einen Bakterium, das verschiedenen Antibiotika ausgesetzt ist. Spektrale Veränderungen der Kultur werden verwendet, um die Reaktionsmechanismen des Bakteriums näher zu erforschen. Ziel der Studie ist es, die Fähigkeit der FT-IR Spektroskopie zu untersuchen, spektrale Veränderungen durch Antibiotika zu erfassen und diese Veränderungen zu erklären. Schließlich wird dargelegt, dass die physiologische Antwort von Mikroorganismen auf Antibiotika studiert werden kann.

In C. KIRSCHNER ET AL.: "FT-IR Spectroscopic Investigations of Antibiotic Sensitive and Resistant Microorgansims", POSTER: 8TH EUROPEAN CONFERENCE ON THE SPECTROSCOPY OF BIOLOGICAL MOLECULES, ENSCHEDE, NIEDERLANDE, wird die Verwendung von FT-IR und NIR Raman Untersuchungen beschrieben, um Spektren von Mikroorganismen zu erfassen. FT-IR wird zur Untersuchung der Antibiotikaempfindlichkeit von Mikroorganismen verwendet, um den Mikroorganismus zu klassifizieren. Lediglich ein einzelnes, bekanntes Antibiotikum wird verwendet, um antibiotikaresistete Organismen von nicht antibiotikaresisteten Organismen zu unterscheiden.

In D. NAUMANN: "Biophysikalische Strukturanalysen", ROBERT KOCH INSTITUT FORSCHUNGSPROJEKTE, PROF. DR. D. NAUMANN, 2000, Seiten 1-4 werden FT-IR Untersuchungen für Mikroorganismen beschrieben, um die Mikroorganismen hinsichtlich Antibiotikaresistenz zu untersuchen. Die Untersuchungen werden zur Analyse der Wirkmechanismen innerhalb der Mikroorganismen verwendet. Die analysierten Wirkmechanismen innerhalb der Mikroorganismen sollen bei der Optimierung von Antibiotika behilflich sein; Ziel der Betrachtungen sind die Wirkmechanismen.

WO 94 11526 A beschreibt die Identifikation von Mikroorganismen mittels UV-Spektroskopie. Zur Untersuchung der Antibiotikaresistenz der Mikroorganismen wird die Wachstumsrate anhand der Ribosomen unter Antibiotikaeinfluss erfasst. Als Antibiotikum wird lediglich ein einzelner Wirkstoff (Rifampin) verwendet, der in seiner Wirkung bereits bekannt ist.

In MASON DAVID J ET AL: "An inexpensive infrared growth sensor array for detection of bacterial antibiotic susceptibility.", FEDERATION OF EUROPEAN MICROBIOLOGICAL SOCIETIES MICROBIOLOGY, Vol. III, Nr. 2-3, 1993, Seiten 251-254, wird die Untersuchung einer Kultur unter Einfluss von bekannten Antibiotika beschrieben. Die Antibiotikaresistenz der Kultur wird anhand von behandelten Kontrollstämmen ermittelt, mit denen die Kultur anhand der Durchlässigkeit für IR-Licht einer einzelnen bestimmten Wellenlänge verglichen wird.

Diese Dokumente des Stands der Technik zeigen somit, dass Kulturen anhand des Kriteriums der Antibiotikaresistenz unterschieden werden können, wobei grundsätzlich gleiche, vorbekannte Antibiotika verwendet werden. Ferner wird beschrieben, durch Antibiotikabehandlung die Wirkungen an den Kulturen näher zu beleuchten, auch hier wird grundsätzlich ein einzelnes, bekanntes Antibiotikum verwendet, um die Studien auf die gleiche Grundlage zu stellen und somit eine Vergleich zu ermöglichten. Alle zitierten Dokumente sind auf die Klassifizierung / Betrachtung von nicht vollständig untersuchten Kulturen ausgereichtet, ein Antibiotikum dient hierbei als Mittel, um eine Reaktion auszulösen und um anhand der Reaktion auf Eigenschaften der untersuchten Kultur schließen zu können.

Neben den erwähnten Methoden der Targetidentifizierung wurden auch erste Ansätze für die Verwendung physikalischer Meßtechniken wie zum Beispiel der FT-IR-Spektroskopie zur Charakterisierung des Wirkmechanismus von antibakteriell wirksamen Substanzen beschrieben (Naumann D. et al. (1991) The characterization of microorganisms by Fouriertransform infrared spectroscopy (FT-IR). In: Modem techniques for rapid microbiological analysis, Nelson W. H.,VCH, S. 43-96, Weinheim; EP 0 457 789 B1). Die Grundlage dieses Verfahrens besteht im spektroskopischen Nachweis der Änderung der Molekülzusammensetzung hervorgerufen durch die Inkubation der Bakterienzelle mit der Prüfsubstanz im Vergleich zu einer nicht behandelten Kontrollkultur. Dieses Verfahren basiert auf einer Auswertung von Banden in Form von Flächenintegralen, die miteinander verglichen werden. Es dient der Interpretation, wo es zu einer molekularen Veränderungen in der Zelle gekommen ist, ohne daraus ein typisches Wirkmuster ableiten zu können. Das Verfahren ist reproduzierbar, in der beschriebenen Form jedoch weder allgemein und einheitlich anwendbar noch automatisierbar. Insbesondere sind z.B. neuartige Wirkmechanismen nicht analysierbar, für die bisher noch keine Inhibitoren als Referenzverbindungen zur Verfügung stehen. Das Verfahren erfordert zudem je nach Wirkmechanismus unterschiedliche, zeitaufwendige Analysen.

Der Erfindung liegt die Aufgabe zugrunde, ein Verfahren zur Charakterisierung und/oder Identifizierung von Wirkmechanismen antimikrobieller Prüfsubstanzen zu entwickeln. Das erfindungsgemäße Verfahren soll schnell sein und eine einheitliche Charakterisierung und/oder Identifizierung unterschiedlicher Wirkmechanismen erlauben und durch Automatisierungsmöglichkeiten auch in der industriellen Forschung und Entwicklung sowie in Routinelaboratorien effektiv einsetzbar sein.

Diese Aufgabe wird gelöst durch das erfindungsgemässe Verfahren zur Charakterisierung einer antimikrobiellen Substanz enthaltend die Schritte
a) Behandeln einer mikrobiellen Kultur mit der zu prüfenden Substanz;
b) Aufnahme von mindestens einem Spektrum (Prüfspektrum) aus der Gruppe von IR-, FT-IR-, Raman- und FT-Raman-Spektren;
c) Vergleich des oder der Prüfspektren aus b) mit einem oder mehreren, in ein, zwei oder mehr Klassen eingeteilte Spektren (Referenzspektren) von mit Referenzsubstanzen behandelten mikrobiellen Kulturen,
d) Zuordnung der Prüfspektren zu einer, zwei oder mehreren der Klassen von Referenzspektren in der Referenzdatenbank.

In einer bevorzugten Ausführungsform der vorliegenden Erfindung erfolgt der Vergleich mit mathematischen Verfahren der Mustererkennung.

In einer weiteren bevorzugten Ausführungsform der vorliegenden Erfindung werden die in Referenzspektren und/oder die Prüfspektren in einer Weise verarbeitet, die eine automatische Erkennung der charakteristischen spektralen Veränderungen und Muster erlaubt.

In einer weiteren bevorzugten Ausführungsform der vorliegenden Erfindung erfolgt die Klassifikation durch eine Mustererkennung, die zwei oder mehr Klassen simultan trennen kann.

In einer weiteren bevorzugten Ausführungsform der vorliegenden Erfindung wird die für eine der Klassen charakteristische Information eines spektralen Musters in einem Klassifikationsmodell oder in Form der Gewichte bei künstlichen neuronalen Neten gespeichert

In einer weiteren bevorzugten Ausführungsform der vorliegenden Erfindung erfolgt der Vergleich der Prüfspektren mit den Referenzspektren durch das Klassifikationsmodell.

Die funktionellen Gruppen aller biochemischen Bestandteile einer mikrobiellen Kultur wie Peptide, Proteine, Polysaccharide, Phospholipide, Nukleinsäuren und Intermediärmetabolite tragen zu einem Spektrum dieser Kultur bei und ergeben einen spezifischen, biochemischen Fingerprint. Die Spektren sind aufgrund der Vielzahl von Komponenten sehr komplex zusammengesetzt und spiegeln viele verschiedene Vibrationsmodi der Biomoleküle der Zellwand, der Cytoplasmamembran, des Cytoplasmas sowie der extrazellulären polymeren Substanzen (z.B. Peptidoglykan, Lipopolysaccharide, (Lipo)-Teichonsäuren) wieder. Die Spektren sind trotz ihrer Komplexität sehr spezifisch für eine Zusammensetzung, Eigenschaft oder einen Zustand einer mikrobiellen Kultur, die bevorzugt eine mikrobielle Reinkultur ist. Da sich die Zusammensetzung, der Zustand und die Eigenschaften von mikrobiellen Kulturen unter dem Einfluß einer Behandlung mit antimikrobiellen Substanzen in spezifischer, substanzabhängiger Weise ändern, kann die spektroskopische Erfassung dieser Änderungen zur Identifizierung und/oder Charakterisierung des Wirkmechanismus herangezogen werden. Bei den Wirkmechanismen kann es sich z.B. um Inhibitoren der Proteinbiosynthese, des RNA- oder DNA-Metabolismus, des Zellwand- oder Lipidstoffwechsels, um membranotrophe Substanzen oder DNA-Interkalatoren handeln. Die genannten Wirkmechanismen sind als beispielhaft und keineswegs erschöpfend anzusehen und von jedem Fachmann in dem Gebiet leicht zu ergänzen.

Durch das erfindungsgemässe Verfahren werden die Vorzüge einer spektroskopischen Messtechnik mit einer dezidierten mathematischen Auswertung des Informationsgehaltes von Spektren verbunden.

Die Referenzdatenbank wird aufgebaut, indem bestimmte mikrobielle Kulturen mit Prüfsubstanzen, deren Wirkmechanismus bekannt ist, bei exakt analoger Einstellung aller Kulturbedingungen wie Temperatur, pH-Wert, Kultivierungsmedium und Zeitdauer, behandelt werden. Von den so behandelten mikrobielle Kulturen werden Referenzspektren aufgenommen und diese in der Referenzdatenbank der Klasse, die zu dem entsprechenden Wirkmechanismus gehört, zugeordnet.

Die einer Klasse zugeordneten Referenzspektren weisen in einem oder mehreren ausgewählten Wellenlängenbereichen eine gleiche oder ähnliche Struktur auf, die sich von der Struktur der Referenzspektren anderer Klassen in den ausgewählten Wellenlängenbereichen signifikant unterscheidet.

Die Auswahl der Wellenlängenbereiche zur Unterscheidung der Klassen ("feature selection") kann durch verschiedene Verfahren wie beispielsweise durch die Varianzanalyse, Kovarianzanalyse, die Faktoranalyse, statistische Distanzmaße wie der euklidischen Distanz oder der Mahalanobis-Distanz oder einer Kombination dieser Methoden mit einem Optimierunsverfahren wie den genetischen Algorithmen erfolgen. Durch die Verwendung oder die Kombination von genetischen Algorithmen kann eine automatisierte und optimierte Suche nach Wellenlängen erfolgen. Hierbei werden die Wellenlängen schneller, effizienter und für die Klassifikation optimal in einem Ranking zusammengestellt. Wesentlich ist dabei, dass eine automatisierte Identifikation der spektralen Bereiche stattfindet, die einen Beitrag an der spektralen Veränderung leisten. Mit diesen identifizierten Bereichen kann ein automatisiertes Klassifikationssystem aufgebaut werden. Meistbevorzugt erfolgt die Auswertung durch eine Kombination von genetischen Algorithmen mit der Kovarianzanalyse.

Vor der Wellenlängenselektion kann eine Vorverarbeitung der Referenzspektren zur Erhöhung des spektralen Kontrastes durch die Bildung von Ableitungen, Dekonvolution, Filterung, Rauschunterdrückung oder Datenreduktion durch Wavelet-Transformation oder Faktorzerlegung erfolgen.

Die Einteilung (Klassifikation) der Referenzspektren in die Klassen in der Referenzdatenbank erfolgt durch mathematische Klassifikationsmethoden wie multivariate, statistische Verfahren der Mustererkennung, neuronale Netze, Methoden des fallbasierten Klassifizierens oder des maschinellen Lernens, genetische Algorithmen oder Methoden des evolutionären Programmierens. Mehrere künstliche neuronale Netze können als feed-forward-Netz mit drei Lagen und einer Gradientenabstiegsmethode als Lernalgorithmus verwendet werden. Das Klassifikationssystem kann eine baumartige Struktur aufweisen, wobei Klassifikationsaufgaben in Teilaufgaben zerlegt werden und die einzelnen Klassifikationssysteme in einer Einheit zu einem hierarchischen Klassifikationssystem zusammengefaßt werden und dabei automatisiert alle hierarchischen Stufen bei der Auswertung durchlaufen werden. Bei den einzelnen Stufen im Klassifikationssystemen kann es sich hierbei um neuronale Netze handeln, die für spezielle Aufgaben optimiert wurden.

Auch eine Kombination von neuronalen Netzen mit einem genetischen Algorithmus ist möglich, um eine Optimierung der Klassifikation durch neuronale Netze vorzunehmen. Die Optimierung kann zum Beispiel durch die Verbesserung der Netzarchitektur oder des Lernalgorithmus erfolgen.

Die Referenzdatenbank kann auch die Form eines künstlichen neuronalen Netzes besitzen, in dem die spektralen Informationen in Form neuronaler Gewichte gespeichert sind und für die Auswertung herangezogen werden können.

Grundsätzlich ist die Erstellung der Referenzdatenbank für die Charakterisierung und/oder Identifizierung der Wirkmechanismen in einer mikrobiellen Kultur nur einmal erforderlich. Darüber hinaus besteht jederzeit die Möglichkeit, die Referenzdatenbank zu erweitern. Dies kann z.B. dadurch geschehen, daß weitere Substanzen in bereits in der Referenzdatenbank enthaltene Klassen von Wirkmechanismen mit aufgenommen werden. Unabhängig davon kann die Referenzdatenbank auch durch weitere bislang nicht enthaltene Klassen von Wirkmechanismen erweitert werden. In diesen Fällen ist ein erneuter Aufbau bzw. eine Ergänzung der Datenbank wie beschrieben erforderlich, wobei diejenigen spektralen Datensätze, die bereits zum Aufbau der vorhergehenden Datenbank verwendeten wurden, nicht neu erhoben werden müssen, solange der verwendete Mikroorganismus, dessen Kulturbedingungen und die spektralen Meßparameter nicht verändert werden.

Die Zuordnung eines Prüfspektrums zu einer, zwei oder mehreren Klassen der Referenzspektren kann durch die mathematischen Klassifikationsmethoden basierend auf der Mustererkennung erfolgen. Insbesondere Methoden, die eine gleichzeitige, simultane Klassifikation in mehrere Klassen zulassen, wie dies etwa bei einer Klassifikation durch künstliche neuronale Netze der Fall ist, sind geeignet für die automatisierte und leistungsfähige Klassifikation mehrerer Klassen. Auch Verfahren basierend auf der Wahrscheinlichkeitsdichtefunktion, der Korrelationsmatrix, Methoden des fallbasierten Klassifizierens oder des maschinellen Lernens, genetische Algorithmen oder Methoden des evolutionären Programmierens sind prinzipiell geeignet. Das Klassifikationssystem kann auch aus mehreren Subeinheiten bestehen, die eine baumartige Struktur aufweisen, wobei Klassifikationsaufgaben in Teilaufgaben zerlegt werden und die einzelnen Klassifikationssysteme in einer Einheit zu einem hierarchischen Klassifikationssystem zusammengefaßt werden und dabei automatisiert alle hierarchischen Stufen bei der Auswertung durchlaufen werden.

Das Prüfspektrum einer Substanz mit unbekanntem Wirkmechanismus wird mit der oder den exakt gleichartigen Kulturen (identischer Mikroorganismenstamm) erstellt, die auch bei der Aufnahme der Referenzdaten eingesetzt werden. Weiterhin müssen alle Kulturbedingungen (beispielsweise Temperatur, pH-Wert, Kultivierungsmedium und Zeitraum) exakt mit denjenigen übereinstimmen, die während der Erstellung der Referenzdatenbank eingehalten werden.

Die Zuordnung eines Prüfspektrums zu einer, zwei oder mehreren Klassen der Referenzspektren kann durch mathematische Klassifikationsmethoden wie multivariate, statistische Verfahren der Mustererkennung, neuronale Netze, Methoden des fallbasierten Klassifizierens oder des maschinellen Lernens, genetische Algorithmen oder Methoden des evolutionären Programmierens erfolgen.

Die Behandlung der mikrobiellen Kultur vor Aufnahme der Spektren kann folgendermassen vor sich gehen:
Die Mikroorganismen (Testkeime) werden in einem geeigneten, mikrobiologischen Nährmedium, das flüssig oder fest sein kann, kultiviert. Dann wird die Prüfsubstanz bzw. Referenzsubstanz mit den Bakterien in Kontakt gebracht. Nach Ablauf einer geeigneten Einwirkungszeit, die vorzugsweise zwischen fünf und 500 Minuten liegt, werden die behandelten Bakterien von der Prüfsubstanz bzw. Referenzsubstanz getrennt z.B. durch Zentrifugation oder Filtration bei Durchführung des Verfahrens in Flüssigkultur bzw. durch Abnahme der Zellen mit Hilfe einer Impföse von einem festen Nährmedium. Zur Entfernung von Resten des Prüfpräparats werden die Zellen in einem geeigneten Volumen einmal, besser jedoch wiederholt gewaschen.

Danach können die Spektren aufgenommen werden. Die Arbeitsschritte der Filtration oder Zentrifugation können auch dadurch umgangen werden, dass eine Messung von Testkeimen mit Prüfsubstanz im Vergleich zu einer unbehandelten Kontrolle der Testkeime erfolgt. Danach wird eine automatisierte Subtraktion der Spektren durchgeführt. Das dadurch erhaltene Spektrum basiert dadurch nur auf den Veränderungen, die durch die Wirksubstanz hervorgerufen werden.

Das erfindungsgemäße Verfahren ist mit IR-, FT-IR-, Raman- und FT-Ramanspektren gleichermaßen durchführbar.

Die Aufnahme von IR-Spektren erfolgt typischerweise im Spektralbereich des sogenannten mittleren Infrarots zwischen 500 - 4000 cm⁻¹, kann aber auch im nahen Infrarotbereich zwischen 4000 und 10.000 cm⁻¹ gemessen oder um diesen Bereich ergänzt werden.

Für die Aufnahme von IR- oder Raman-Spektren kann jede der an sich bekannten spektroskopischen Meßanordnungen eingesetzt werden wie Transmission/Absorption, abgeschwächte Totalreflexion, direkte oder diffuse Reflexion oder IR-Lichtleitertechnik. Besonders vorteilhaft ist die Messung in Transmission/Absorption.

Die Proben der mikrobiologischen Kultur sind vorzugsweise fest oder flüssig. Bei der Messung ist der Einsatz von Multiküvetten für das Messen mehrerer Proben oder der Einsatz mikrospektrometrischer Techniken vorteilhaft. Dazu zählen die FT-IR- und Raman-, FT-Raman-Mikroskopie oder andere Vefahren der Strahlfokussierung. Dadurch kann eine Minimierung der Probenmenge erreicht werden und der Einsatz einer automatisierten Probenvorverarbeitung und -messung erfolgen, um so den Probendurchsatz zu erhöhen und eine Etablierung für das High-Throughput-Screening zu ermöglichen. Es können auch Probenträger wie sie für Mikrotiterplatten verwendet werden oder Durchflußküvetten, benutzt werden. Auch der Einsatz von Durchflußküvetten, gekoppelt mit einem automatiserten HPLC-Probenaufgabesystem würde einen erhöhten Probendurchsatz ermöglichen. Es ist auch der Einsatz von Infrarotlichtleitern für eine ortsunabhängigere Meßautomatisierung möglich.

Als Material für Küvetten und Probenträger der oben beschriebenen Präparationsvarianten können alle in der IR-Spektroskopie üblicherweise verwendeten wasserunlöslichen optischen Materialien eingesetzt werden wie Ge, ZnSe, CaF₂, BaF₂, wobei sich für ein Multiprobenelement ZnSe bewährt hat. Aber auch aufgeraute Metallplatten oder Mikrometallgitter sind als Probenhalter geeignet, besonders wenn sie im Maßstab der Mikrotiterplatten für eine Vielzahl von Proben und als Einwegmaterialien konzipiert sind.

Die Probenmenge für die Aufnahme von IR-Spektren kann sehr klein gehalten werden und nur wenige µl (2-5 µl) betragen. Je nach den vorgegebenenen Bedingungen mit oder ohne Strahlfokussierung können Substanzmengen von µg bis ng eingesetzt werden. Die Durchmesser der durchstrahlten Probenareale variieren von 1 bis 6 mm, im Fall einer Mikrofukussierung 5 bis 50 µm.

Im Falle von Raman-Messungen bietet sich auch eine Messung in flüssiger Kultur an, die direkt in den Gefäßen der Probenvorbereitung erfolgen kann, wie z.B. Mikrotiterplatten. Dadurch kann ein erheblicher Zeitvorteil und höherer Automatisierungsgrad erreicht werden, da dadurch Ablaufzeiten verkürzt werden und Probenvorbereitungsschritte entfallen. Durch die Verwendung einer konfokalen Strahlführung kann eine optimierte Positionierung des Ramansignals erfolgen, um Störsignale zu eliminieren und das Signalzu-Rausch-Verhältnis zu verbessern.. Eine Anordnung von simultan verwendeten Lichtquellen oder eine entsprechende Vervielfältigung der Anregungslichtes und Lenkung auf die Probe für die Ramanmessung sowie die Verwendung von parallel eingesetzten Detektoren (z.B. CCDs) kann den Probendurchsatz und die Automatisierbarkeit zusätzlich wesentlich erhöhen.

Die Prüfsubstanz kann ein Hemmstoff sein. Die Konzentration an Hemmstoff, mit dem die Bakterienkultur behandelt wird, liegt vorzugsweise im Bereich des 0,1- bis 20-fachen der minimalen Hemmstoffkonzentration (MHK) für die Prüfsubstanz. Die minimalen Hemmstoffkonzentration (MHK) ist die minimale Konzentration eines Antibiotikums, mit der ein Testkeim in seinem Wachstum über 18-24 h inhibiert wird. Die Hemmstoffkonzentration kann dabei nach mikrobiologischen Standardverfahren bestimmt werden (siehe z.B. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000.). Die Prüfspektren werden von einer mikrobiellen Kultur aufgenommen, die jeweils mit dem Hemmstoff entweder in einer, vorzugsweise aber in mehreren unterschiedlichen Konzentrationen behandelt wurde.

Das erfindungsgemässe Verfahren ist zur Untersuchung unterschiedlichster Zellkulturen geeignet. Eine bevorzugte Gruppe von Kulturen sind mikrobielle Zellkulturen wie Bakterien, Pilzen, Hefen, Archaebakterien und dergleichen. Die Erfindung umfasst aber auch die Untersuchung von Zellkulturen nicht-mikrobieller Herkunft, beispielsweise Krebszellen, immunologisch funktionellen Zellen, Epithelzellen, Pflanzenzellen und dergleichen. Somit sind erfindungsgemäss sowohl Anwendungen im Bereich der funktionellen Zellcharakterisierung als auch der Bereich toxikologischer Untersuchungen abgedeckt.

Das erfindungsgemässe Verfahren zeichnet sich dadurch aus, dass es empfindlich, standardisierbar und reproduzierbar ist. Es ist allgemein und einheitlich auf verschiedenste Wirkmechanismen anwendbar. Es ist kostengünstig und liefert schnelle Ergebnisse.

Ein weiterer Vorteil des erfindungsgemässen Verfahrens besteht in der Möglichkeit der Einbeziehung von Mutanten des verwendeten Testkeims, wobei die Mutation zu einer Unterexpression eines bestimmten Targets führt und auf diese Weise die Inhibierung dieses Targets durch einen potentiellen Inhibitor nachstellt. Solche Mutanten können nach dem Stand der Technik heute leicht für jedes beliebige Target hergestellt werden (Guzman L.M. et al. (1995) Tight regulation, modulation, and high-level expression by vectors containing the arabinose PBAD promoter. J. Bacteriol. 177(14):4121-30). Auf diese Weise kann somit auch der Mechanismus von Hemmstoffen auf solche Targets bestimmt werden, für die noch keine Referenz - Inhibitoren bekannt sind.

### Figuren und Beispiele

### Beispiel

### Bestimmung der Minimalen Hemmkonzentration (MHK)

Zur Herstellung einer Übernachtkultur wurden 22 ml Belitsky Minimalmedium (Stuhlke et al. (1993) Temporal activation of beta-glucanase synthesis in Bacillus subtilis is mediated by the GTP pool. J. Gen. Microbiol. 1993 Sep; 139 (Pt 9):2041-5) mit einem Aliquot des Testkeims *Bacillus subtilis* 168 aus einer bei -80°C gelagerten Dauerkultur beimpft und bei 37°C, 200 rpm bebrütet. Die Kultur, die nach 16-18 h eine OD₅₀₀ von 1,0-1,6 aufwies, wurde mit Belitsky Minimalmedium auf eine OD₅₀₀ von 0,01 verdünnt (entspricht einer Keimzahl von etwa 0,8-2x10⁵ Keimen pro ml) und im 96er-Mikrotiter-Platten-Maßstab 1:1 mit den im gleichen Medium vorgelegten zu testenden Präparaten inkubiert, welche in seriellen 1:2 Verdünnungen vorlagen. Als MHK wurde die niedrigste Konzentration eines Inhibitors angegeben, bei der nach 18-24 h Inkubation bei 37°C kein bakterielles Wachstum zu beobachten war. In Tabelle 1 sind die MHK-Werte der für die Erstellung der Referenzdatenbank verwendeten Referenzsubstanzen angegeben.

**Tabelle 1: Referenzsubstanzen, MHK-Werte gegen B. subtilis 168 und die eingesetzten Konzentrationen**

| **Referenzsubstanzen** | **MHK [µg/ml]** | **eingesetzte Konzentration [µg/ml]** | | | |
|---|---|---|---|---|---|
| **Tetracyclin** | **16** | **4** | **16** | **64** | |
| **Chloramphenicol** | **4** | **1** | **4** | **16** | |
| **Methicillin** | **0,125** | **0,03** | **0,06** | **0,125** | |
| **Rifampicin** | **0,25** | **0,06** | **0,125** | **0,25** | **1** |
| **Ciprofloxacin** | **0,25** | **0,06** | **0,25** | **0,5** | |
| **Moxifloxacin** | **0,125** | **0,03** | **0,125** | **0,25** | |
| **Kanamycin** | **0,5** | **0,125** | **0,5** | **1** | |
| **Oxacillin** | **0,5** | **0,06** | **0,125** | **0,25** | |
| **Cefoxitin** | **2** | **0,25** | **0,5** | **1** | |
| **Moxalactam** | **4** | **1** | **4** | **8** | |
| **Erythromycin** | **0,5** | **0,125** | **0,5** | **2** | |
| **Fusidinsäure** | **0,5** | **0,125** | **0,5** | **2** | |
| **Nalidixinsäure** | **32** | **8** | **32** | **128** | |
| **Novobiocin** | **2** | **0,5** | **2** | **8** | |
| **Trimethoprim** | **0,5** | **0,125** | **0,5** | **2** | |
| **Vancomycin** | **0,25** | **0,06** | **0,25** | **0,5** | |
| **D-Cycloserin** | **64** | **4** | **8** | **16** | |
| **Clindamycin** | **2** | **0,5** | **2** | **8** | |
| **Gentamicin** | **0,125** | **0,02** | **0,03** | **0,06** | |
| **Penicillin G** | **4** | **1** | **4** | **16** | |
| **Neomycin** | **0,125** | **0,03** | **0,125** | **0,5** | |
| **Tobramycin** | **0,0625** | **0,02** | **0,06** | **0,125** | |
| **Mupirocin** | **0,0625** | **0,02** | **0,06** | **0,25** | |
| **Puromycin** | **8** | **2** | **8** | **16** | |
| **Ristocetin** | **0,5** | **0,125** | **0,5** | **1** | |
| **Teichoplanin** | **0,125** | **0,03** | **0,125** | **0,25** | |
| **Spectinomycin** | **16** | **4** | **16** | **64** | |
| **Streptomycin** | **128** | **16** | **32** | **64** | |
| **Clarithromycin** | **0,0625** | **0,02** | **0,06** | **0,25** | |
| **Azithromycin** | **1** | **0,25** | **1** | **4** | |
| **Oxazolidinon BAY 11-5845** | **0,25** | **0,06** | **0,25** | **1** | |
| **Mitomycin C** | **0,25** | **0,03** | **0,06** | **0,125** | |
| **Mersacidin** | **16** | **1** | **2** | **4** | |
| **Ramoplanin** | **1** | **0,0625** | **0,125** | **0,25** | |
| **Actinomycin D** | **1** | **0,25** | **1** | **4** | |
| **Monesin** | **4** | **1** | **4** | **16** | |
| **Gramicidin S** | **1** | **0,125** | **0,25** | **0,5** | |
| **Gramicidin A** | **4** | **0,0313** | **0,0625** | **0,125** | |
| **Lasalocid** | **1** | **0,25** | **1** | **4** | |
| **Nigericin** | **1** | **0,03** | **0,06** | **0,125** | |
| **Nitrofurantoin** | **16** | **2** | **4** | **8** | |
| **Ethidiumbromid** | **4** | **1** | **4** | **16** | |
| **Proflavin** | **8** | **2** | **8** | **32** | |
| **Cerulenin** | **16** | **4** | **8** | **16** | **64** |
| **Doxorubicin** | **8** | **1** | **2** | **4** | |
| **Azaserin** | **4** | **1** | **4** | **8** | |
| **Enniatin** | **16** | **4** | **8** | **16** | |
| **5-Fluoruracil** | **0,25** | **0,0625** | **0,25** | **1** | |
| **5-Fluor-2-desoxyuridin** | **0,25** | **0,0625** | **0,25** | **1** | **4** |
| **Polymyxin B-sulfat** | **16** | **2** | **4** | **8** | **16** |

### Anzucht der Zellen und Behandlung mit den Referenz- bzw. Prüfsubstanzen

Ausgehend von einer Übernachtkultur, deren Herstellung wie oben beschrieben erfolgte, wurden jeweils 50 ml auf 37°C vorgewärmtes Belitsky Minimalmedium mit je 1 ml der Übernachtkultur angeimpft und bei 37°C mit 200 Umdrehungen inkubiert. In der exponentiellen Wachstumsphase bei OD₅₀₀ 0,25 - 0,27 wurden die Substanzen in den in Tabelle 1 angegebenen Konzentrationen zugesetzt und die Ansätze für weitere 150 min inkubiert. Als Kontrolle wurde zu jedem Experiment eine unbehandelte Kultur in Einfachbestimmung mitgeführt. Zur Erfassung interner Varianzen wurde jedes Präparat bei jeder Konzentration in einer Fünffachbestimmung getestet. Die eingesetzten Konzentrationen wurden im Vorfeld anhand eines Wachstumsexperiments so ausgewählt, dass nach 150 min Einwirkzeit zwar ein Effekt auf die Wachstumsgeschwindigkeit im Vergleich zu einer nicht behandelten Kontrollkultur sichtbar wurde, jedoch noch keine lytischen Prozesse - weder in der Wachstumskurve noch nach mikroskopischer Untersuchung - eingesetzt hatten.

### Aufarbeitung der Proben für die FT-IR spektroskopische Untersuchung

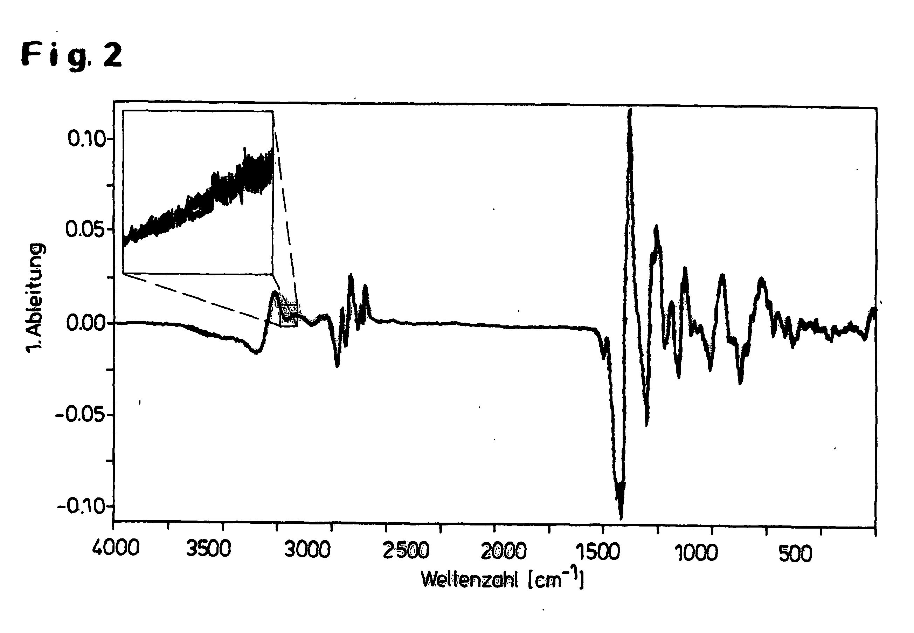

Nach Behandlung der Bakterienzellen für 150 min mit den Referenz- bzw. Prüfsubstanzen wurden je 20 ml der Kulturen in einer Heraeus Sepatech Minifuge T bei 5500 x g (5650 rpm) für 10 min bei 16°C abzentrifugiert. Die Zellsedimente wurden zweimal mit je 1 ml Wasser gewaschen, wobei die Zellen zwischen den Waschschritten in einer Eppendorf-Zentrifuge bei 13000 rpm für 10 min sedimentiert wurden. Die Proben wurden schließlich so in Wasser aufgenommen und sorgfältig resuspendiert, dass sich nach der anschließenden 30 minütigen Trocknung von je 35 µl Zellen bei 40-50 mbar bei Raumtemperatur unter P₄O₁₀ homogene Bakterienfilme bildeten, deren Absorption im Bereich von 0,345 bis 1,245 Absorptionseineinheiten (AU) lag.
Die FT-IR Spektren der mit den Prüfsubstanzen behandelten Bakterienkulturen wurden mit Hilfe eines IFS 28B FT-IR Spektrometers (Bruker, Ettlingen) im Absorptionsmodus aufgenommen mit einem ZnSe Probenhalter für 15 Probenpositionen. Die Spektren wurden mit einem DTGS-Detektor und 64 Scans im Wellenlängenbereich von 4000 - 500 cm⁻¹ aufgezeichnet. Die Fourier-Transformation wurde mit einer Blackman-Harris 3-Term Apodisationsfunktion sowie einem zero-filling Faktor von 4 zur Erreichung einer spektralen Auflösung von 6 cm⁻¹ durchgeführt.
Zur Minimierung von Artefakten durch Wasserdampf in der Raumluft wurde das Spektrometer permanent mit 500-1000 1/h trockener Luft, die mit Hilfe eines Zander Lufltrockners hergestellt wurde, geflutet. Der Wasserdampfgehalt wurde während der Aufnahme der Spektren im Bereich von 1837 bis 1847 cm⁻¹ gemessen und betrug nicht mehr als 0,0003 AU.
Unter diesen Bedingungen betrug das Rauschen nicht mehr als 0,0003 AU im Bereich von 2000 bis 2100 cm⁻¹.
Ein Test zur Qualitätskontrolle der gemessenen FT-IR Spektren wurde auf die Spektren angewendet mit Schwellenwerten für minimale Absorption (0.345 AU) und maximale Absorption (1.245 AU), was im Linearitätsbereich des Detektors liegt.
Vor jeder Messung an einer Probe wurde ein Hintergrundspektrum aufgenommen, um so den Hintergrund zu kompensieren.
Zu jeder Probe wurden 5 unabhängige Messungen durchgeführt, um Varianzen von Messung zu Messung für jede Probe mit aufzunehmen. Die Reproduzierbarkeit der Spektrenaufnahme über einen Zeitraum von sechs Monaten ist in Abb. 2 dargestellt. Die Steuerung des Spektrophotometers erfolgte durch die Optics User Software OPUS 3.0 (Version 970717.0) von Bruker, Ettlingen, Deutschland.
Die im folgenden beschriebenen mathematischen Verfahren zur Datenauswertung wurden zur Erhöhung des spektralen Kontrastes auf die FT-IR-Spektren nach Bildung der ersten Ableitung unter Verwendung eines Savitzki-Golay Algorithmus (Savitzky A. and Golay M.J. (1964) Smoothing and differentiation of data by simplified least square procedures. Anal. Chem. 36: 1627-1638) unter Berücksichtigung von 9 Glättungspunkten und Durchführung einer Vektornormierung angewandt.

### Erstellung des mathematischen Klassifikationsmodells:

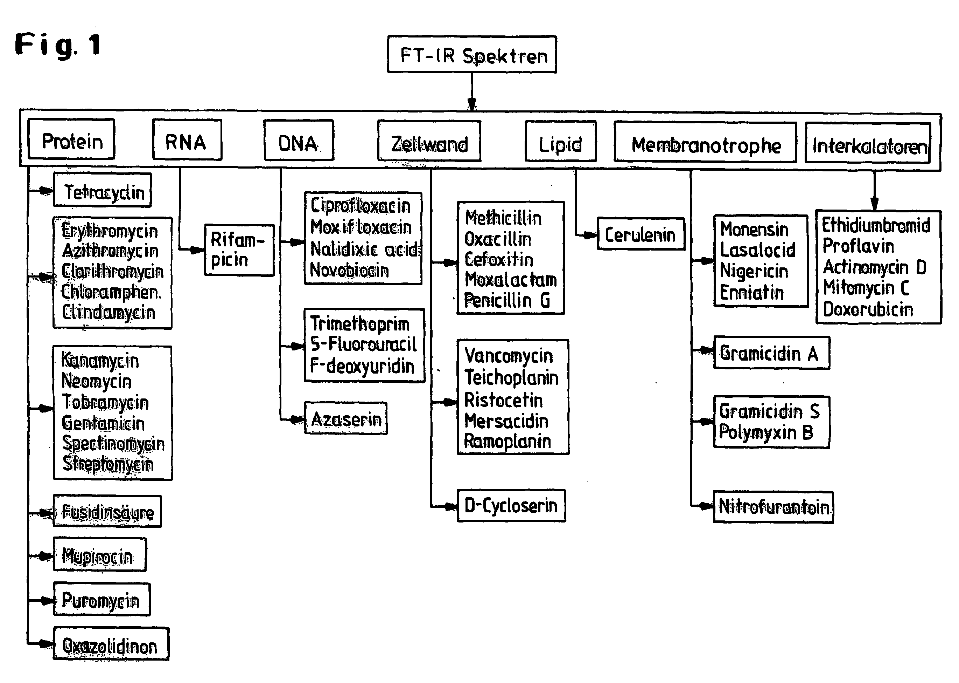

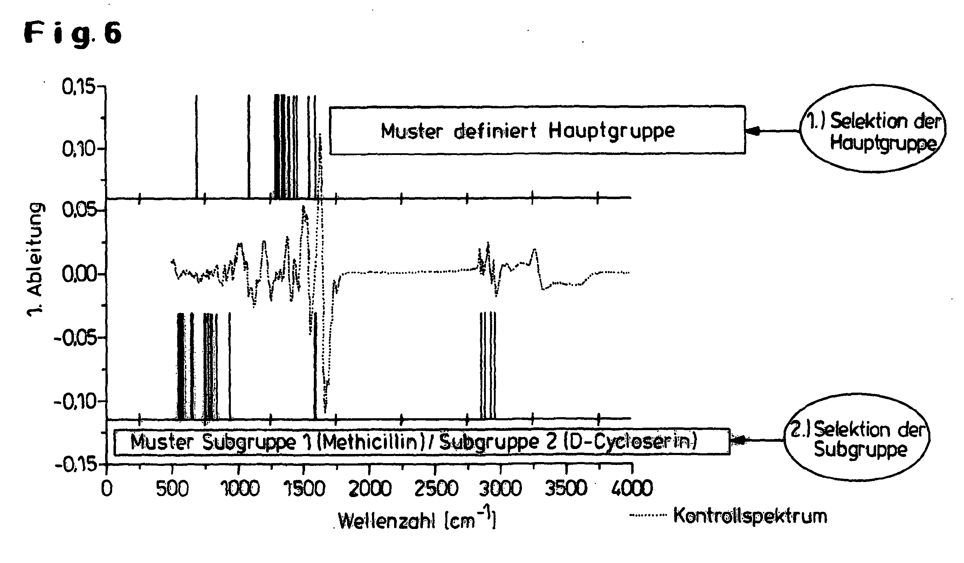

Für die Erstellung des mathematischen Klassifikationsmodells wurden die Referenzspektren nach Bildung der 1. Ableitung zugrunde gelegt. Danach wurde eine Normierung zum Zwecke der spektralen Vergleichbarkeit hinsichtlich der Intensitäten mittels einer Vektornormierung durchgeführt (OPUS Software Manual S. 126, Firma Bruker Ettlingen). Danach wurde eine Einteilung der Referenzdaten in die gewünschte Zahl von unterschiedlichen Wirkmechanismen vorgenommen, in diesem Beispiel eine Anzahl von 7 Hauptgruppen (siehe Abb. 1). Die Referenzspektren wurden nach ihrer Zugehörigkeit zu diesen 7 Hauptgruppen sortiert. Ziel dieser Sortierung ist es, mit den mathematischen Verfahren die Wellenlängen heraus zu finden, die sich für eine Klassifikation der spektralen Muster der einzelnen Gruppen besonders gut eignen (feature selection). Ein Verfahren zur Wellenlängenselektion, das zum Einsatz kam, berechnet die euklidische Distanz eines jeden spektralen Datenpunktes und das Centroid (Klassenmittelpunkt) für jede Wellenlänge. Für die Klassifikation sind die Wellenlängen besonders geeignet, deren euklidische Distanz innerhalb der Klassen (zum Centroid) möglichst klein ist, deren Abstand zwischen den unterschiedlichen Klassen aber möglichst maximal ist. Eine automatisierte und optimierte Suche nach Wellenlängen, die diese Kriterien besitzen, erfolgte mit einem genetischen Algorithmus. Hierbei werden die Wellenlängen schneller, effizienter und für die Klassifikation optimal in einem Ranking zusammengestellt. Aus dieser Liste mit Wellenlängen geordnet nach ihrem Klassifikationspotential wurden später die Wellenlängen für das Modell zur Klassifikation mit neuronalen Netzen gewählt.
Ein zweiter Ansatz stützte sich auf die Berechnung der Varianzen (univariat und covariat) eines jeden Datenpunktes der Referenzspektren innerhalb der Gruppe, die mit der Varianz zwischen den Gruppen verglichen wurde. Anschließend wurde ein automatisches Ranking der Wellenlängen durchgeführt, bei dem die Varianz innerhalb der Gruppe möglichst klein ist und zwischen den verschiedenen Gruppen möglichst groß ist. Die besten 97 Wellenlängen wurden aus diesem Ranking als Input-Neuronen für ein neuronales Netz verwendet. Die Wellenlängenwahl nach diesem Verfahren ist in Fig. 6 dargestellt.
Als Klassifikationsmodell kam ein drei-lagiges feedforward-Netz zum Einsatz mit 97 Input-Neuronen, 22 Hidden-Neuronen und 7 Output-Neuronen. Als Lernalgorithmus wurde der Resilient-Backpropagation-Algorithmus (RProp) verwendet. Die Ausgabeaktivierungen wurden zwischen 0 und 1 gesetzt.

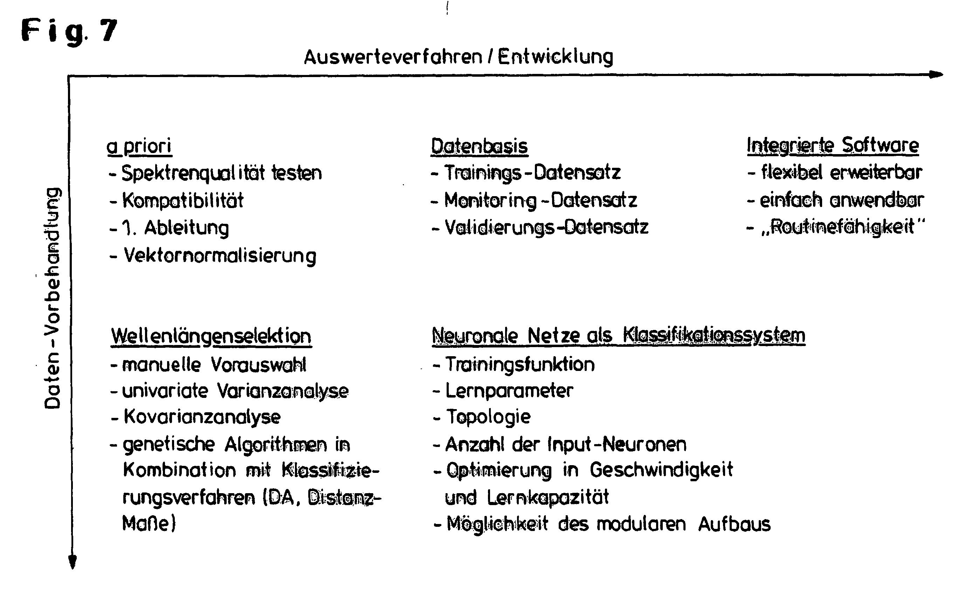

Fig. 7 zeigt die Konzeption der Datenverarbeitung.

### Klassifikation einer Substanz X mit unbekanntem Wirkmechanismus:

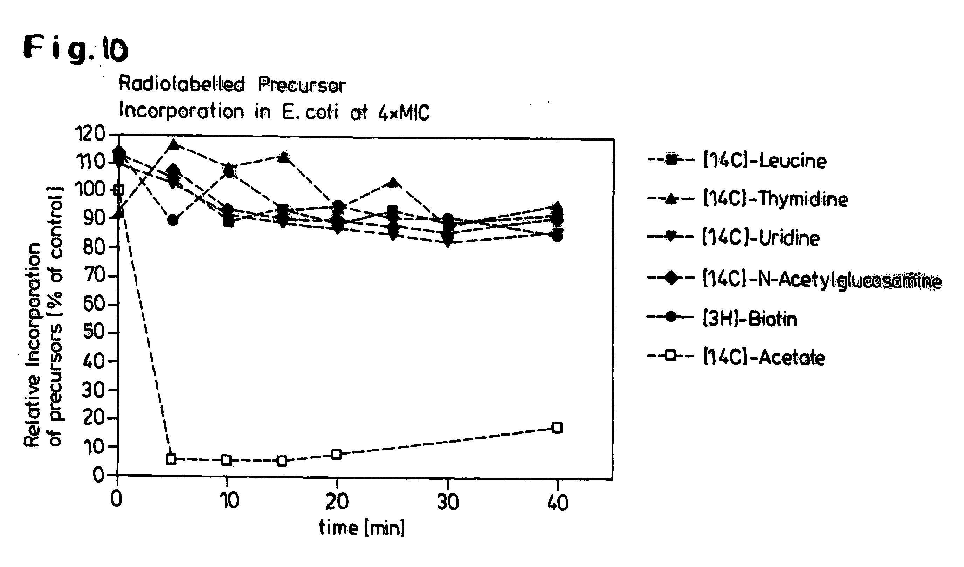

Zur externen Validierung des beschriebenen Verfahrens wurden die Bakterienzellen mit der antibakteriell wirksamen Substanz X (MHK 2 µg/ml) in Fünffachbestimmung in den Konzentrationen 1, 2 und 4 µg/ml behandelt. Die Durchführung des Klassifikationsverfahrens ergab bei Behandlung mit 2 und 4 µg/ml in allen Fällen eine eindeutige Zuordnung der Spektren in die Klasse der mit Cerulenin behandelten Proben. Bei Cerulenin handelt es sich um einen Inhibitor des Fettsäurebiosynthese-Stoffwechsels, was die Vermutung nahelegt, dass die Substanz X einen ähnlichen Wirkmechanismus wie Cerulenin aufweist. In der Tat zeigt Fig. 10, dass die Substanz X selektiv den *de novo* Einbau von [¹⁴C]-Acetat in CHCl₃/MeOH extrahierbare Phospholipide inhibiert. Die Auswertung der Spektren der mit nur 1µg/ml der Substanz X behandelten Bakterien ergab keinerlei Zuordnung, was möglicherweise aufgrund der niedrigen Dosierung an den nur sehr geringen Änderungen in der Wachstumskurve und im FT-IR-Spektrum im Vergleich zu den unbehandelten Kontrollkulturen liegen könnte.

Die Figuren zeigen
- Fig. 1: Struktur der Referenzdatenbank auf Basis der Wirkmechanismen bekannter Antibiotika
- Fig. 2: Reproduzierbarkeit der Messung der Spektren
- Fig. 3: Differenzierung von Antibiotikaklassen
- Fig. 4: Spektren von Proteinbiosyntheseinhibitoren
- Fig. 5: Verfahren zur Wellenlängenselektion
- Fig. 6: Hierarchische Zuordnung des Wirkmechanismus
- Fig. 7: Konzeption der Datenverarbeitung
- Fig. 8: Beispiel für Wirkmechanismus von Substanz X
- Fig. 9: Auswertung von Spektrum von Substanz X in einem 1. Wellenlängenbereich
- Fig. 10: Auswertung von Spektrum von Substanz X in einem 2. Wellenlängenbereich
- Fig. 11: Beispiel für Wirkmechanismus von Substanz Y
- Fig. 12: Auswertung von Spektrum von Substanz Y

Fig. 1 zeigt den Aufbau des für das Beispiel verwendeten Klassifizierungssystems in Form hierarchischer neuronaler Netze zusammen mit der Zuordnung der Referenzantibiotika. In einem ersten Klassifizierungsschritt werden die 7 Hauptklassen der Inhibitoren (Proteinbiosynthese-Inhibitoren, RNA-Biosynthese-Inhibitoren DNA-Biosynthese-Inhibitoren Zellwand-Biosynthese-Inhibitoren, Lipid-Biosynthese-Inhibitoren, Membranotrope-Substanzen, Interkalatoren) voneinander getrennt. In einen zweiten Schritt werden dann Untergruppen unterschieden (Bsp. DNA-Biosyntheseinhibitoren mit den 3 Untergruppen 1.Ciprofloxacin-ähnliche Substanzen, 2. Trimethoprim-ähnlicheSubstanzen, 3. Azaserin-ähnliche Substanzen. Diese Aufteilung in Untergruppen ist vom Prinzip her fortführbar und erweiterbar. Die getroffenen Zuordnungen sind für den Fachmann in dem Gebiet unmittelbar nachvollziehbar und können aus einschlägigen Referenzwerken (z.B. Graefe U. (1992) Biochemie der Antibiotika, pp. 15-39, Spektrum Akademischer Verlag Heidelberg, Berlin, New York) erstellt werden.

Fig. 2 zeigt die Überlagerung der 1. Ableitung von 25 zufällig ausgewählten Spektren des Mikroorganismus *Bacillus subtilis* Stamm 168 ohne Zusatz eines Hemmstoffs. Die Spektren sind über einen Zeitraum von 6 Monaten aufgenommen worden. Alle 25 Spektren sind praktisch identisch und weisen nur eine vernachlässigbare Varianz auf. Das zeigt die gute Reproduzierbarkeit der Aufnahme von Spektren von mikrobiellen Kulturen. Die Reproduzierbarkeit ist eine wichtige Voraussetzung für das Gelingen des erfindungsgemässen Verfahrens.

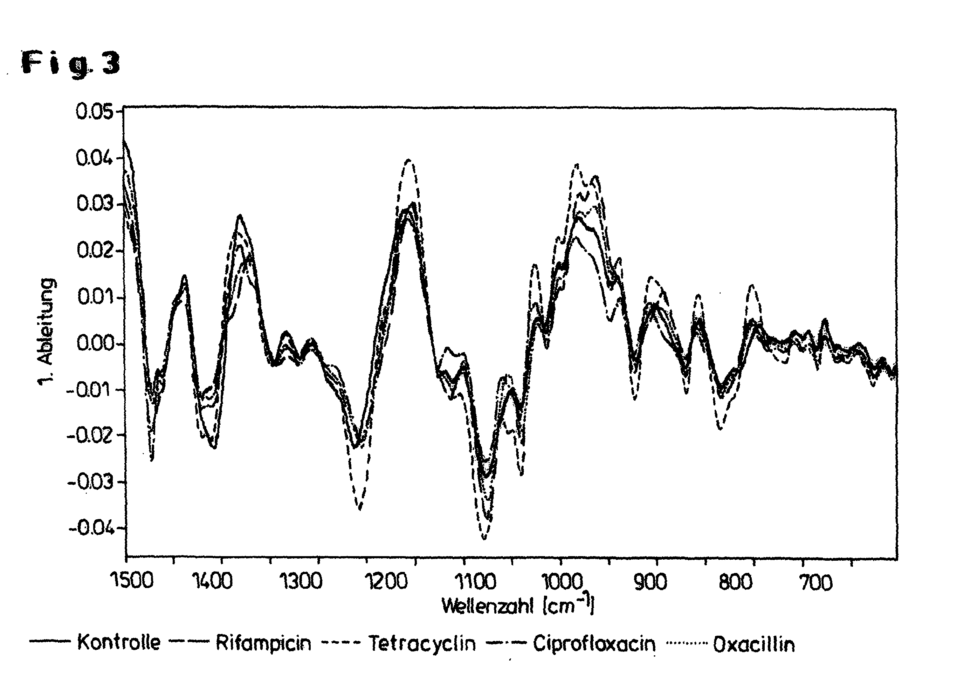

Fig. 3 zeigt die 1. Ableitung von 25 in unabhängigen Experimenten erhobenen Kontrollspektren einer Bakterienkultur von *Bacillus subtilis* Stamm 168 ohne Behandlung mit einer Prüfsubstanz und darüber überlagert jeweils 5 mal die 1. Ableitung von Spektren von Bakterienkulturen desselben Stamms, die mit den verschiedenen Antibiotika Rifampicin, Tetracyclin, Ciprofloxacin und Oxacillin behandelt worden sind. Den verschiedenen Antibiotika sind gemäss Fig. 1 verschiedene Wirkmechanismen zugeordnet. Dementsprechend unterscheiden sich auch die Spektren der bakteriellen Kulturen, die mit den unterschiedlichen Antibiotika behandelt worden sind. Die Einwirkzeit betrug jeweils 150 min, die Konzentration war die 4 fache minimale Hemmkonzentration (MHK) bzw. 0,25 fache MHK im Fall von Tetracyclin. Die MHK-Werte der Antibiotika betragen 0,25 µg/ml für Rifampicin, 16 µg/ml für Tetracyclin, 0,25 µg/ml für Ciprofloxacin sowie 0,5 µg/ml für Oxacillin.

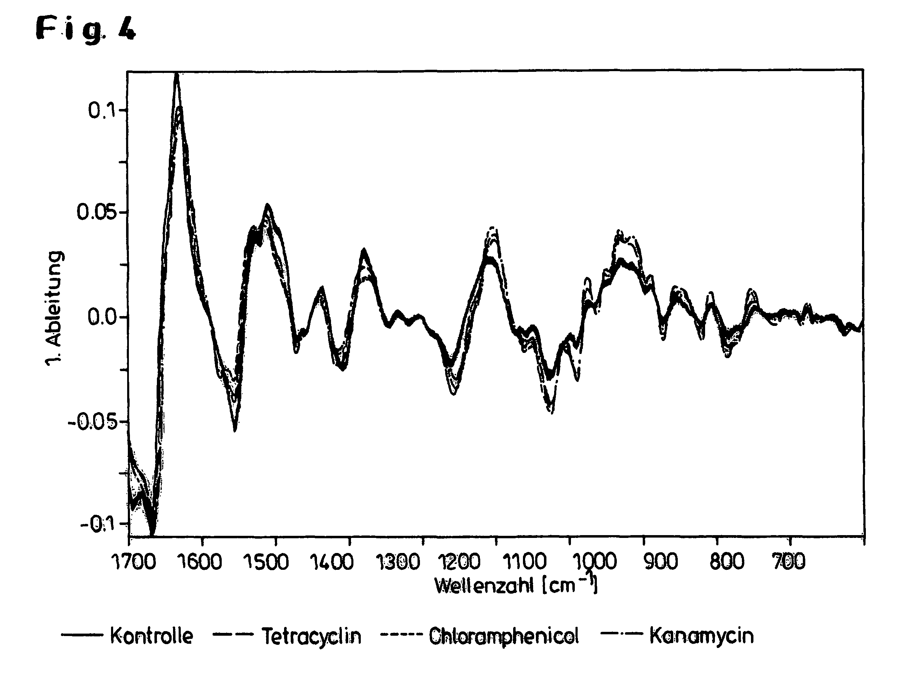

Fig. 4 zeigt die 1. Ableitung von 25 Kontrollspektren einer Bakterienkultur ohne Behandlung mit einer Prüfsubstanz und darüber überlagert jeweils 5 mal die 1. Ableitung von Spektren von Bakterienkulturen, die mit den verschiedenen Antibiotika Tetracyclin (4 µg/ml), Chloramphenicol (4 µg/ml) und Kanamycin (4 µg/ml) behandelt worden sind. Die Behandlungsdauer der Bakterienkulturen betrug jeweils 150 min. Bei allen drei hier getesteten Antibiotika handelt es sich um Proteinbiosyntheseinhibitoren. Die 1. Ableitungen der Spektren der mit diesen verschiedenen Proteinbiosyntheseinhibitoren behandelten Spektren weisen eine gute Übereinstimmung untereinander und signifikante Unterschiede zu den 1. Ableitungen der Kontrollspektren auf.

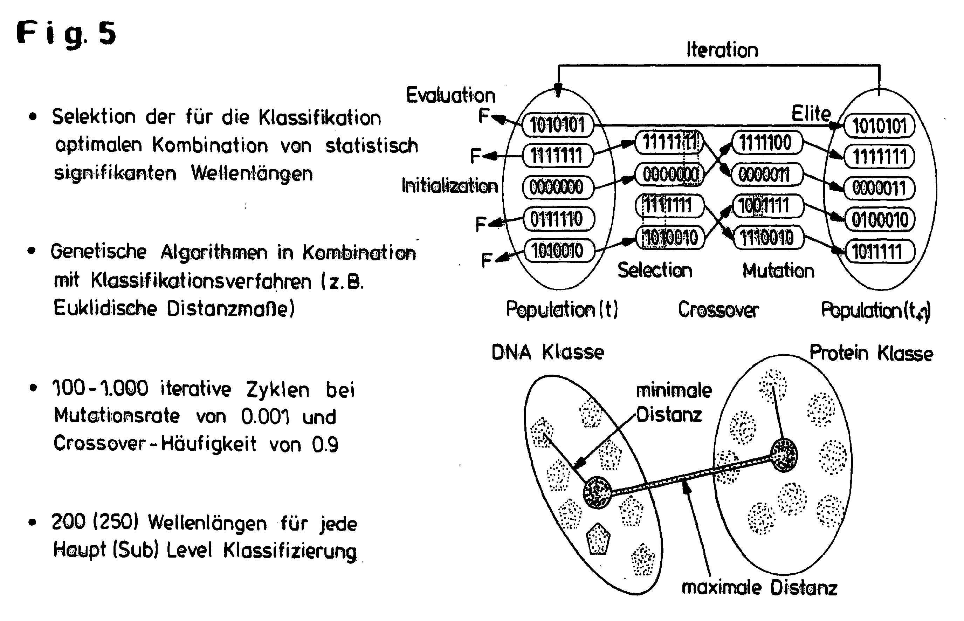

Fig. 5 erläutert ein Beispiel für ein Verfahren zur Wellenlängenselektion. Hierbei wird die Berechnung der euklidischen Distanz eines jeden spektralen Datenpunktes durchgeführt und das Centroid (Klassenmittelpunkt) für jede Wellenlänge berechnet. Für die Klassifikation sind die Wellenlängen besonders geeignet, deren euklidische Distanz innerhalb der Klassen (zum Centroid) möglichst klein ist, deren Abstand zwischen den unterschiedlichen Klassen aber möglichst maximal ist. Eine automatisierte und optimierte Suche nach Wellenlängen, die diese Kriterien besitzen erfolgt mit einem genetischen Algorithmus. Hierbei werden die Wellenlängen schneller, effizienter und für die Klassifikation optimal in einem Ranking zusammengestellt. Aus dieser Liste mit Wellenlängen geordnet nach ihrem Klassifikationspotential werden später die Wellenlängen für ein Modell zur Klassifikation gewählt (z.B. neuronale Netze).

Fig. 6 zeigt die hierarchische Zuordnung des Wirkmechanismus. Die schwarzen Balken repräsentieren diejenigen Wellenlängenbereiche, die für die Klassifikation der Antibiotika entsprechend ihren Wirkmechanismen herangezogen werden. Im oberen Teil der Abbildung sind die spektralen Bereiche dargestellt, die eine besonders hohe Signifikanz für die Trennung der 7 Hauptgruppen (Inhibitoren der Protein- RNA-, DNA-, Lipid- und Zellwand-Synthese, sowie membranotrophe Substanzen und Interkalatoren) aufweisen; der untere Teil der Abbildung stellt dagegen die Spektralbereiche dar, die für die Klassifikation der Antibiotika in verschiedene Subgruppen innerhalb einer Hauptgruppe am Beispiel der Trennung von β-Lactamen und D-Cycloserin innerhalb der Hauptgruppe Zellwandsyntheseinhibitoren genutzt werden.

Fig. 7 zeigt die Konzeption der Datenverarbeitung.

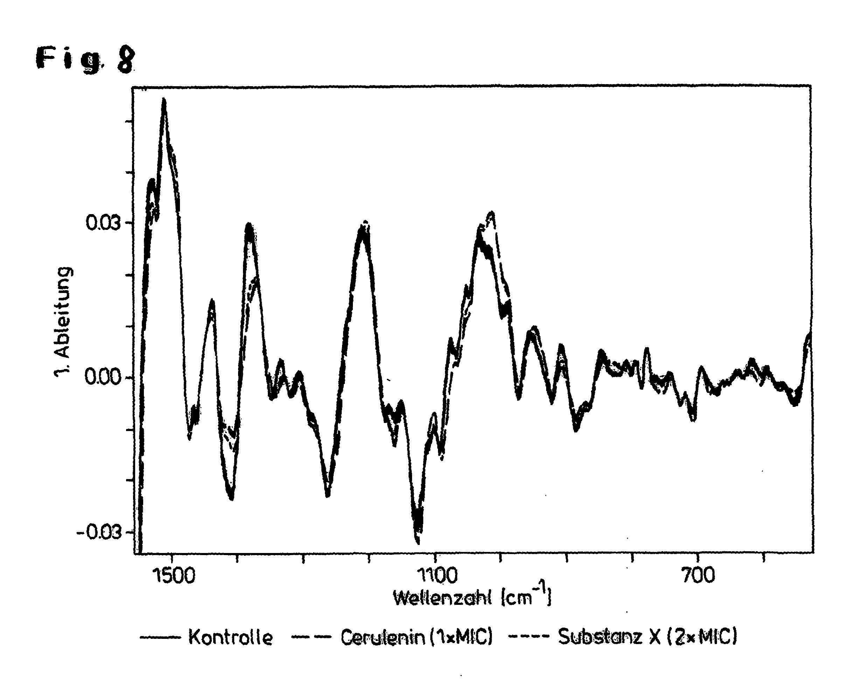

Fig. 8 zeigt den Wirkmechanismus einer Substanz X. Substanz X inhibiert selektiv den *de novo* Einbau von [¹⁴C]-Acetate in CHCl₃/MeOH extrahierbare Phospholipide.

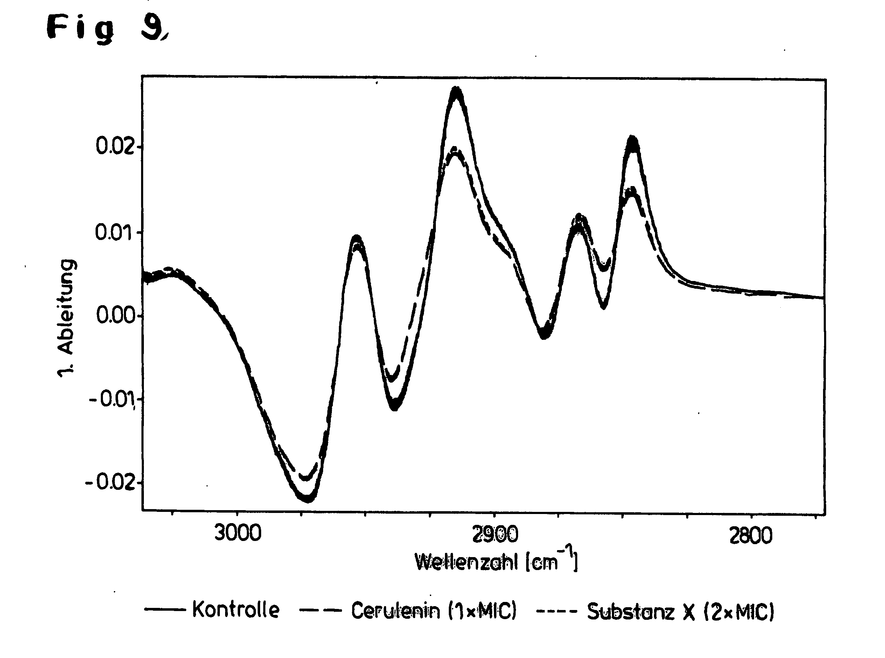

Fig. 9 zeigt die 1. Ableitung von 25 Kontrollspektren einer Bakterienkultur ohne Behandlung mit einer Prüfsubstanz und darüber überlagert jeweils 5 mal die 1. Ableitung von Spektren von Bakterienkulturen, die mit Cerulenin (1 fach MHK; 16 µg/ml) und der Substanz X (2 fach MHK; 2 µg/ml) behandelt worden sind. Wie Fig. 1 zu entnehmen ist handelt es sich bei Cerulenin um einen Lipidsyntheseinhibitor. Die Ähnlichkeit im FT-IR Muster deutet darauf hin, dass es sich bei der unbekannten Prüfsubstanz X ebenfalls um eine Inhibition der Lipidsynthese handelt. Fig. 10 zeigt dieselben Spektren wie Fig. 9 in einem anderen Wellenlängenbereich. Dieser Spektralbereich wird dominiert von Vibrationsübergängen der Fettsäuremoleküle. In diesem Spektralbereich sind die Unterschiede zwischen den Referenzspektren und den Prüfspektren mit den lipidsyntheseinhibierenden Prüfsubstanzen besonders signifikant.

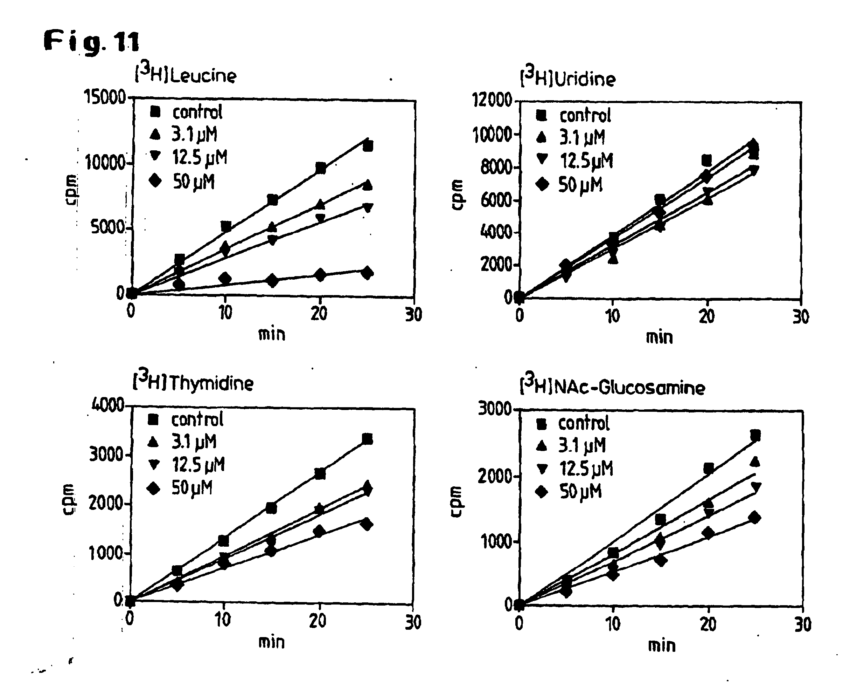

Fig. 11 zeigt den Wirkmechanismus einer Substanz Y. Substanz Y inhibiert selektiv den *de novo* Einbau von [³H]-Leucin in Perchlorsäure präzipitierbares Material.

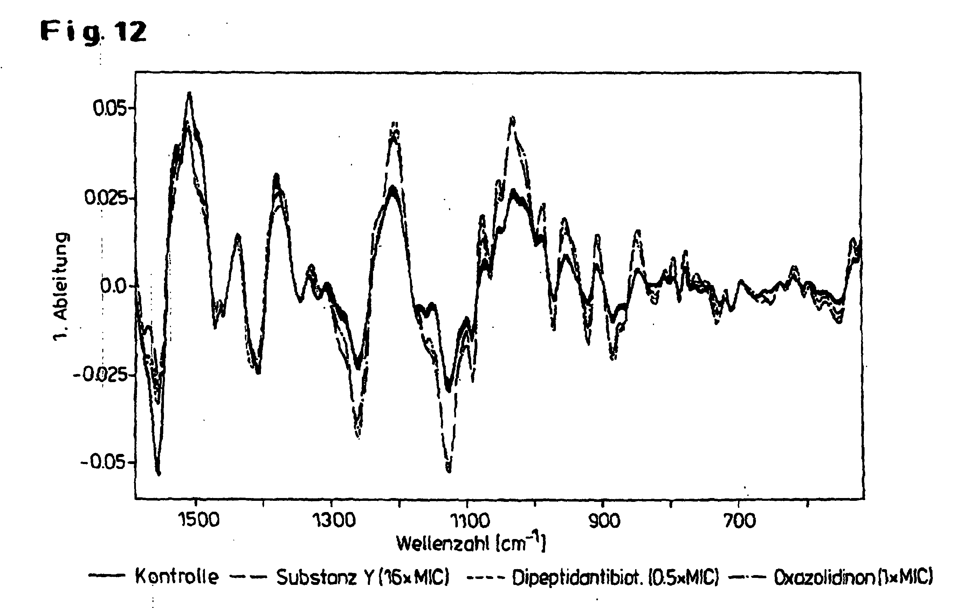

Fig. 12 zeigt die 1. Ableitung eines Kontrollspektrums einer Bakterienkultur ohne Behandlung mit einer Prüfsubstanz und darüber überlagert die 1. Ableitung von Spektren von Bakterienkulturen, die mit einem Dipeptidantibiotikum (0,5 fach MHK; 0,5 mg/L), einem Oxazolidinon (1 fach MHK; 2 mg/L) und der Substanz Y (16 fach MHK, 3 mg/L) behandelt worden sind. Wie Fig. 1 zu entnehmen ist handelt es sich bei dem Oxazolidinon um einen Proteinbiosyntheseinhibitor, gleiches gilt für das Dipeptidantibiotikum. Die Ähnlichkeit im IR Muster deutet darauf hin, dass es sich bei der unbekannten Prüfsubstanz Y ebenfalls um eine Inhibition der Proteinbiosynthese handelt.

## Patentansprüche

1. Verfahren zur Identifizierung oder Charakterisierung des Wirkmechanismus einer antimikrobiellen Substanz enthaltend die Schritte
a) Erstellen von Referenzspektren durch Behandlung bestimmter mikrobieller Kulturen mit Prüfsubstanzen, deren Wirkmechanismus bekannt ist, und Aufnahme mindestens eines Spektrums aus der Gruppe von IR-, FT-IR-, Raman-und FT-Raman-Spektren
b) jeweils Auswahl von mindestens einem Wellenlängenbereich gleicher oder ähnlicher Struktur zur Unterscheidung der dem entsprechenden Wirkmechanismus zugehörenden Klassen, und Einteilung der Referenzspektren in die Klassen in der Referenzdatenbank, wobei die einer Klasse zugeordneten Referenzspektren in dem ausgewählten Wellenlängenbereich eine gleiche oder ähnliche Struktur aufweisen, die sich von der Struktur der Referenzspektren anderer Klassen in dem ausgewählten Wellenlängenbereich signifikant unterscheidet
c) Behandeln einer mikrobiellen Kultur mit der zu prüfenden Substanz
d) Aufnahme von mindestens einem Spektrum (Prüfspektrum) aus der Gruppe von IR, FT-IR-, Raman- und FT-Raman-Spektren;
e) Vergleich des oder der Prüfspektren aus d) mit einem oder mehreren Referenzspektren in der Referenzdatenbank,
f) Zuordnung der Prüfspektren zu einer, zwei oder mehreren der Klassen von Referenzspektren in der Referenzdatenbank und Identifizierung oder Charakterisierung des Wirkmechanismus.

2. Verfahren nach Anspruch 1, **dadurch gekennzeichnet, dass** der Vergleich e) mit mathematischen Verfahren der Mustererkennung erfolgt.

3. Verfahren nach Anspruch 1 oder 2, **dadurch gekennzeichnet, dass** die in Schritt d) erhaltenen Spektren in einer Weise verarbeitet werden, die eine automatische Erkennung der charakteristischen spektralen Veränderungen und Muster erlaubt.

4. Verfahren nach einem der Anspruche 1 bis 3, **dadurch gekennzeichnet, dass** die Klassifikation durch eine Mustererkennung erfolgt, die zwei oder mehr Klassen simultan trennen kann.

5. Verfahren nach einem der Ansprüche 1 bis 4, **dadurch gekennzeichnet, dass** die für eine der Klassen charakteristische Information eines spektralen Musters in einem Klassifikationsmodell oder in Form der Gewichte bei künstlichen neuronalen Netzen gespeichert wird.

6. Verfahren nach einem der Ansprüche 1 bis 5, **dadurch gekennzeichnet, dass** der Vergleich der Prüfspektren mit den Referenzspektren durch das Klassifikationsmodell erfolgt.

7. Verfahren nach einem der Ansprüche 1 bis 6, **dadurch gekennzeichnet, dass** die mikrobielle Kultur eine Reinkultur ist.

8. Verfahren nach einem der Ansprüche 1 bis 7, **dadurch gekennzeichnet, dass** es sich bei dem Wirkmechanismus um Inhibitoren der Proteinbiosynthese, des RNA- oder DNA-Metabolismus, des Zellwand- oder Lipidstoffwechsels, um membranotrophe Substanzen oder DNA-Interkalatoren handelt.

9. Verfahren nach einem der Ansprüche 1 bis 8, **dadurch gekennzeichnet, dass** für die Bereitstellung der Referenzdatenbank zusätzlich definierte Mutanten des mikrobiellen Keims genutzt werden, vorzugsweise solche mit erniedrigter oder erhöhter Produktion eines ausgewählten Zielgens oder solche mit eingeschränkter oder erhöhter biologischer Aktivität auf Grund von Punktmutationen und/oder Deletionen, wobei die Mutation des betreffenden Zielgens die Interaktion des Genprodukts mit einer hypothetischen Referenzsubstanz nachstellt

10. Verfahren nach einem der Ansprüche 1 bis 9, **dadurch gekennzeichnet, dass** die Auswahl der Wellenlängenbereiche zur Unterscheidung der Klassen (Wellenlängenselektion) durch multivariate statistische Verfahren wie die Varianzanalyse, Kovarianzanalyse, die Faktoranalyse, statistische Distanzmaße wie der euklidischen Distanz oder der Mahalanobis-Distanz oder einer Kombination dieser Methoden mit einem Optimierunsverfahren wie den genetischen Algorithmen erfolgt.

11. Verfahren nach einem der Ansprüche 1 bis 10, **dadurch gekennzeichnet, dass** vor der Wellenlängeselektion eine Vorverarbeitung der Referenzspektren zur Erhöhung des spektralen Kontrastes durch die Bildung von Ableitungen, Dekonvolution, Filterung, Rauschunterdrückung oder Datenreduktion durch Wavelet-Transformation oder Faktorzerlegung erfolgt.

12. Verfahren nach einem der Ansprüche 1 bis 11, **dadurch gekennzeichnet, dass** die Einteilung der Referenzspektren in die Klassen in der Referenzdatenbank durch mathematische Klassifikationsmethoden der Mustererkennung, einem Allgemeinen Linearen Modell, durch, künstliche neuronale Netze, Methoden des fallbasierten Klassifizierens, der Vektor-Optimierung oder des maschinellen Lernens, genetische Algorithmen oder Methoden des evolutionären Programmierens erfolgt.

13. Verfahren nach einem der Ansprüche 1 bis 12, **dadurch gekennzeichnet, dass** die Einteilung der Referenzspektren in die Klassen in der Referenzdatenbank durch mathematische Klassifikationsmethoden wie multivariate, statistische Verfahren der Mustererkennung, neuronale Netze, Methoden des fallbasierten Klassifizierens oder des maschinellen Lernens, genetische Algorithmen oder Methoden des evolutionären Programmierens erfolgt.

14. Verfahren nach Anspruch 13, **dadurch gekennzeichnet, dass** mehrere künstliche neuronale Netze und Klassifikationsmethoden verwendet werden.

15. Verfahren nach Anspruch 14, **dadurch gekennzeichnet, dass** mehrere künstliche neuronale Netze als feed-forward-Netz mit drei Lagen und einer Gradientenabstiegsmethode als Lernalgorithmus verwendet werden.

16. Verfahren nach einem der Ansprüche 14 oder 15, **dadurch gekennzeichnet, dass** das Klassifikationssystem eine baumartige Struktur aufweist, wobei Klassifikationsaufgaben in Teilaufgaben zerlegt werden und die einzelnen Klassifikationssysteme in einer Einheit zu einem hierarchischen Klassifikationssystem zusammengefaßt werden und dabei automatisiert alle hierarchische Stufen bei der Auswertung durchlaufen werden.

17. Verfahren nach Anspruch 16, **dadurch gekennzeichnet, dass** es sich bei den einzelnen Klassifikationssystemen um auf spezielle Aufgaben optimierte neuronale Netze handelt.

18. Verfahren nach einem der Ansprüche 1 bis 17, **dadurch gekennzeichnet, dass** die Zuordnung eines Prüfspektrums zur einer, zwei oder mehreren Klassen der Referenzspektren durch mathematische Klassifikationsmethoden wie multivariate, statistische Verfahren der Mustererkennung, neuronale Netze, Methoden des fallbasierten Klassifizierens oder des maschinellen Lernens, genetische Algorithmen oder Methoden des evolutionären Programmierens erfolgt.

19. Verfahren nach einem der Ansprüche 1 bis 18, **dadurch gekennzeichnet dass** die Aufnahme von IR-Spektren im Spektralbereich von 500 bis 4000 cm⁻¹ und/oder von 4000 bis 10.000 cm⁻¹ erfolgt.

20. Verfahren nach einem der Ansprüche 1 bis 19, **dadurch gekennzeichnet, dass** die Prüfsubstanz ein Hemmstoff ist.

21. Verfahren nach einem der Ansprüche 1 bis 20, **dadurch gekennzeichnet, dass** die Konzentration an Hemmstoff, mit dem die Bakterienkultur behandelt wird, im Bereich des 0,1- bis 20-fachen der minimalen Hemmstoffkonzentration für die Prüfsubstanz liegt.

22. Verfahren nach einem der Ansprüche 1 bis 21, **dadurch gekennzeichnet, dass** Prüfspektren von einer mikrobiellen Kultur aufgenommen werden die jeweils mit demselben Hemmstoff aber in unterschiedlicher Konzentration behandelt wurde.

23. Verfahren nach einem der Ansprüche 1 bis 22, **dadurch gekennzeichnet, dass** die Messung in Küvetten, Durchflussldivetten und Mikroküvetten erfolgt, die in Transmission, Absorption oder Reflektion vermessen werden und für automatisierte Messungen/Durchfluβmessungen und das High-Throuhput-screening geeignet sind.

24. Verfahren nach einem der Ansprüche 1 bis 23, **dadurch gekennzeichnet, dass** FT-IR, IR, Raman und FT -Raman-Messungen direkt in Flüssigkeiten und Gefäßen der Probenauf- und -vorbereitung gemessen werden können.

25. Verfahren nach einem der Ansprüche 1 bis 24, **dadurch gekennzeichnet, dass** als mikrobielle Zellkulturen pro- oder eukaryontische Zellen eingesetzt werden, vorzugsweise Bakterien, Pilzen, Hefen, oder Archaebakterien.

## Claims

1. Process for identifying or characterising the active mechanism of an antimicrobial substance, including the following steps:
a) creating reference spectra by treatment of certain microbial cultures with test substances, the active mechanism of which is known, and recording of at least one spectrum from the group comprising IR, FT-IR, Raman and FT-Raman spectra
b) in each case, selection of at least one wavelength region of the same or similar structure for the purpose of distinguishing the classes pertaining to the corresponding active mechanism, and classification of the reference spectra into the classes in the reference database, whereby the reference spectra assigned to a class exhibit in the selected wavelength region an identical or similar structure which differs significantly from the structure of the reference spectra of other classes in the selected wavelength region
c) treating a microbial culture with the substance to be tested
d) recording of at least one spectrum (test spectrum) from the group comprising IR, FT-IR, Raman and FT-Raman spectra
e) comparison of the test spectrum/spectra from d) with one or more reference spectra in the reference database
f) assignment of the test spectra to one, two or more of the classes of reference spectra in the reference database, and identification or characterisation of the active mechanism.

2. Process according to Claim 1, **characterised in that** the comparison e) is undertaken using mathematical methods of pattern recognition.

3. Process according to Claim 1 or 2, **characterised in that that** the spectra obtained in step d) are processed in a manner that permits an automatic recognition of the characteristic spectral changes and patterns.

4. Process according to one of Claims 1 to 3, **characterised in that** the classification is undertaken by a pattern recognition that is able to distinguish between two or more classes simultaneously.

5. Process according to one of Claims 1 to 4, **characterised in that** the information of a spectral pattern that is characteristic of one of the classes is stored in a classification model or in the form of the weights in artificial neural networks.

6. Process according to one of Claims 1 to 5, **characterised in that** the comparison of the test spectra with the reference spectra is undertaken by the classification model.

7. Process according to one of Claims 1 to 6, **characterised in that** the microbial culture is a pure culture.

8. Process according to one of Claims 1 to 7, **characterised in that** in the case of the active mechanism it is a question of inhibitors of protein biosynthesis, of RNA or DNA metabolism, of cell-wall or lipid metabolism, membranotrophic substances or DNA intercalators.

9. Process according to one of Claims 1 to 8, **characterised in that** for the provision of the reference database additionally defined mutants of the microbial germ are utilised, preferentially those with decreased or increased production of a selected target gene or those with limited or increased biological activity by reason of point mutations and/or deletions, whereby the mutation of the target gene in question adjusts the interaction of the gene product with a hypothetical reference substance.

10. Process according to one of Claims 1 to 9, **characterised in that** the selection of the wavelength regions for the purpose of distinguishing the classes (wavelength selection) is undertaken by multivariate statistical methods such as variance analysis, covariance analysis, factor analysis, statistical distance measures such as the Euclidean distance or the Mahalanobis distance or a combination of these methods with an optimisation method such as genetic algorithms.

11. Process according to one of Claims 1 to 10, **characterised in that** prior to the wavelength selection a preprocessing of the reference spectra is undertaken for the purpose of increasing the spectral contrast through the formation of derivatives, deconvolution, filtering, noise suppression or data reduction by wavelet transformation or factorisation.

12. Process according to one of Claims 1 to 11, **characterised in that** the classification of the reference spectra into the classes in the reference database is undertaken by mathematical classification methods of pattern recognition, a general linear model, by artificial neural networks, methods of case-based classifying, of vector optimisation or of machine learning, genetic algorithms or methods of evolutionary programming.

13. Process according to one of Claims 1 to 12, **characterised in that** the classification of the reference spectra into the classes in the reference database is undertaken by mathematical classification methods such as multivariate statistical methods of pattern recognition, neural networks, methods of case-based classifying or of machine learning, genetic algorithms or methods of evolutionary programming.

14. Process according to Claim 13, **characterised in that** use is made of several artificial neural networks and classification methods.

15. Process according to Claim 14, **characterised in that** use is made of several artificial neural networks by way of feed-forward network with three layers and with a gradient-descent method by way of learning algorithm.

16. Process according to one of Claims 14 or 15, **characterised in that** the classification system exhibits a tree-like structure in which classification tasks are broken down into partial tasks and the individual classification systems are combined in a unit to form a hierarchical classification system and thereby all the hierarchical stages are run through in automated manner in the course of the evaluation.

17. Process according to Claim 16, **characterised in that** in the case of the individual classification systems it is a question of neural networks that have been optimised for special tasks.

18. Process according to one of Claims 1 to 17, **characterised in that** the assignment of a test spectrum to one, two or more classes of the reference spectra is undertaken by mathematical classification methods such as multivariate statistical methods of pattern recognition, neural networks, methods of case-based classifying or of machine learning, genetic algorithms or methods of evolutionary programming.

19. Process according to one of Claims 1 to 18, **characterised in that** the recording of IR spectra is undertaken in the spectral region from 500 cm⁻¹ to 4000 cm⁻¹ and/or from 4000 cm⁻¹ to 10,000 cm⁻¹.

20. Process according to one of Claims 1 to 19, **characterised in that** the test substance is an inhibiting agent.

21. Process according to one of Claims 1 to 20, **characterised in that** the concentration of inhibiting agent with which the bacterial culture is treated lies within the range from 0.1 times to 20 times the minimal inhibiting-agent concentration for the test substance.

22. Process according to one of Claims 1 to 21, **characterised in that** test spectra are recorded of a microbial culture which has been treated in each case with the same inhibiting agent but in differing concentration.

23. Process according to one of Claims 1 to 22, **characterised in that** the measurement is undertaken in cells, flow cells and microcells which are measured in transmission, absorption and reflection and are suitable for automated measurements or flow measurements and for high-throughput screening.

24. Process according to one of Claims 1 to 23, **characterised in that** FT-IR, IR, Raman and FT-Raman measurements can be measured directly in liquids and vessels pertaining to sample preparation.

25. Process according to one of Claims 1 to 24, **characterised in that** prokaryotic or eukaryotic cells - preferentially bacteria, moulds, yeasts or archaebacteria - are employed as microbial cell cultures.

## Revendications

1. Procédé d'identification ou de caractérisation du mécanisme d'action d'une substance anti-microbienne comprenant les étapes consistant à :
a) produire des spectres de référence par traitement de certaines cultures microbiennes avec des substances de test dont le mécanisme d' action est connu, et à enregistrer au moins un spectre du groupe comprenant les spectres IR, FT-IR, Raman et FT-Raman,
b) choisir respectivement au moins une plage de longueur d'ondes de structure identique ou similaire pour distinguer les classes appartenant au mécanisme d'action correspondant, et la répartition des spectres de référence dans les classes dans la banque de données de référence, les spectres de références affectés à une classe présentant dans leur plage de longueurs d'ondes choisies, une structure identique ou similaire qui se distingue significativement de la structure des spectres de référence d'autres classes dans la plage de longueur d'ondes choisie,
c) traiter une culture microbienne avec la substance à tester,
d) enregistrer au moins un spectre (spectre de test) du groupe des spectres IR, FT-IR, Raman et FT-Raman ;
e)comparer le ou les spectres de test du point d) avec un ou plusieurs spectres de référence dans la banque de données de référence ;
f) affecter les spectres de test à une, deux classes de spectres de référence ou plus dans la banque de données de référence et identifier ou caractériser le mécanisme d'action.

2. Procédé selon la revendication 1, **caractérisé en ce que** la comparaison e) s'effectue avec le procédé mathématique de reconnaissance de formes.

3. Procédé selon la revendication 1 ou 2, **caractérisé en ce que** les spectres obtenus à l'étape d) sont traités d'une manière qui permet une reconnaissance automatique des modifications spectrales caractéristiques.

4. Procédé selon l'une des revendications 1 à 3, **caractérisé en ce que** la classification s'effectue par une reconnaissance de formes qui peut séparer simultanément deux classes ou plus.

5. Procédé selon l'une des revendications 1 à 4, **caractérisé en ce que** les informations d'une forme spectrale caractéristiques pour l'une des classes sont enregistrées dans un modèle de classification ou sous la forme de poids par des réseaux neuronaux artificiels.

6. Procédé selon l'une des revendications 1 à 5, **caractérisé en ce que** la comparaison des spectres de test s'effectue avec les spectres de référence par le modèle de classification.

7. Procédé selon l'une des revendications 1 à 6, **caractérisé en ce que** la culture microbienne est une culture pure.

8. Procédé selon l'une des revendications 1 à 7, **caractérisé en ce que** le mécanisme d'action est constitué par des inhibiteurs de la biosynthèse des protéines, du métabolisme de l'ARN ou de l'ADN, du métabolisme de la paroi cellulaire ou du métabolisme des lipides, des substances membranetropes ou des éléments intercalaires d'ADN.

9. Procédé selon l'une des revendications 1 à 8, **caractérisé en ce que**, pour la mise à disposition de la banque de données de référence, on utilise en outre des mutants définis du germe microbien, de préférence, ceux présentant une production réduite ou accrue d'un gène cible choisi ou ceux présentant une activité biologique limitée ou accrue en raison de mutations et/ou délétions ponctuelles, la mutation du gène cible concerné retardant l'interaction du produit de gène avec une substance de référence hypothétique.

10. Procédé selon l'une des revendications 1 à 9, **caractérisé en ce que** le choix des plages de longueurs d'ondes pour distinguer les classes (sélection des longueurs d'ondes) s'effectue par des procédés statistiques multivariés tels que l'analyse de la variance, l'analyse de la covariance, l'analyse factorielle, la mesure de distance statistique telle que la distance euclidienne ou la distance de Mahalanobis ou une combinaison de ces méthodes avec un procédé d'optimisation tel que les algorithmes génétiques.

11. Procédé selon l'une des revendications 1 à 10, **caractérisé en ce que**, avant le choix de la longueur d'ondes s'effectue un prétraitement des spectres de références pour augmenter le contraste spectral par la formation de dérivation, de déconvolution, de filtrage, de suppression du bruit ou de réduction des données par transformation en ondelettes ou de décomposition factorielle.

12. Procédé selon l'une des revendications 1 à 11, **caractérisé en ce que** la répartition des spectres de référence dans les classes de la banque de données de référence s'effectue par des méthodes de classification mathématique par reconnaissance de formes, un modèle linéaire général, par des réseaux neuronaux artificiels, des méthodes de classification à base de cas, d'optimisation par vecteur ou par apprentissage mécanique, algorithmes génétiques ou méthodes de programmation évolutionnaires.

13. Procédé selon l'une des revendications 1 à 12, **caractérisé en ce que** la répartition des spectres de référence dans les classes de la banque de données de référence s'effectue par des méthodes de classification mathématique tels que des procédés statistiques multivariés de reconnaissance de formes, des réseaux neuronaux, des méthodes de classification à base de cas ou d'apprentissage mécanique, d'algorithmes génétiques ou des méthodes de programmation évolutionnaires.

14. Procédé selon la revendication 13, **caractérisé en ce que** l'on utilise plusieurs réseaux neuronaux artificiels et méthodes de classification.

15. Procédé selon la revendication 14, **caractérisé en ce que** l'on utilise plusieurs réseaux neuronaux artificiels en tant que réseau feed-forward avec trois couches et une méthode de diminution de gradient en tant qu'algorithme d'apprentissage.

16. Procédé selon l'une des revendications 14 ou 15, **caractérisé en ce que** le système de classification présente une structure en arbre, la tâche de classification étant décomposée en tâches partielles et les systèmes de classification individuels étant regroupés en une unité pour un système de classification hiérarchique et ainsi, toutes les étapes hiérarchiques étant automatisées lors de l'évaluation.

17. Procédé selon la revendication 16, **caractérisé en ce que** les systèmes de classification individuels sont des tâches spécifiques de réseaux neuronaux optimisés.

18. Procédé selon l'une des revendications 1 à 17, **caractérisé en ce que** l'affectation d'un spectre de test à une, deux classes de spectres de référence ou plus s'effectue par des méthodes de classification mathématique tels qu'un procédé statistique multivarié de reconnaissance de formes, réseaux neuronaux, méthodes de classification à base de cas ou apprentissage mécanique, algorithmes génétiques ou méthodes de programmation évolutionnaires.

19. Procédé selon l'une des revendications 1 à 18, **caractérisé en ce que** l'enregistrement des spectres IR s'effectue dans la plage spectrale de 500 à 4000 cm⁻¹ et/ou de 4000 à 10 000 cm-¹.

20. Procédé selon l'une des revendications 1 à 19, **caractérisé en ce que** la substance de test est un inhibiteur.

21. Procédé selon l'une des revendications 1 à 20, **caractérisé en ce que** la concentration d' inhibiteur avec lequel la culture bactérienne est traitée est de l'ordre de 0,1 à 20 fois la concentration inhibitrice minimale de la substance de test.

22. Procédé selon l'une des revendications 1 à 21, **caractérisé en ce que** les spectres d'essai d'une culture microbienne qui sont traités respectivement avec le même inhibiteur mais en concentration différentie, sont enregistrés.

23. Procédé selon l'une des revendications 1 à 22, **caractérisé en ce que** la mesure s'effectue en cuvettes, cuvettes d'écoulement et microcuvettes, qui sont mesurées en transmission, absorption ou réflexion et sont appropriées pour les mesures/mesures de débit automatiques et le criblage à haut rendement.

24. Procédé selon l'une des revendications 1 à 23, **caractérisé en ce que** les mesures FT, IR, IR, Raman et FT-Raman peuvent être mesurées directement dans les liquides ou les récipients de traitement et préparation d'échantillon.

25. Procédé selon l'une des revendications 1 à 24, **caractérisé en ce que** l'on utilise comme cultures cellulaires microbiennes, des cellules pro- ou eucaryotes, de préférence, des bactéries, des champignons, des levures ou des archae.
